# EUROPÄISCHE PATENTSCHRIFT

(11) **EP 1 272 481 B1**
(45) Veröffentlichungstag und Bekanntmachung des Hinweises auf die Patenterteilung: **21.04.2004**
(21) Anmeldenummer: 01915345.1
(22) Anmeldetag: 15.03.2001
(51) Int. Cl.: C07D 311/60, A61K 31/35, A61P 5/00

(54) **4-FLUORALKYL-2H-BENZOPYRANE MIT ANTIESTROGENER WIRKSAMKEIT**
4-FLUOROALKYL-2H-BENZOPYRANS WITH ANTI-ESTROGENIC ACTIVITY
4-FLUOROALKYL-2H-BENZOPYRANNES A ACTIVITE ANTI-OEOESTROGENIQUE

(30) Priorität: 15.03.2000 DE 10013782
(43) Veröffentlichungstag der Anmeldung: 08.01.2003
(73) Patentinhaber: SCHERING AKTIENGESELLSCHAFT, 13342 Berlin (DE)
(72) Erfinder: KÜNZER, Hermann, 13347 Berlin (DE); JAUTELAT, Rolf, 10439 Berlin (DE); ZORN, Ludwig, 13509 Berlin (DE); HEGELE-HARTUNG, Christa, 45470 Mülheim a.d. Ruhr (DE); KOLLENKIRCHEN, Uwe, 12209 Berlin (DE); FRITZEMEIER, Karl-Heinrich, 13505 Berlin (DE)
(86) Internationale Anmeldenummer: PCT/EP2001/002928
(87) Internationale Veröffentlichungsnummer: WO 2001/068634

(56) Entgegenhaltungen:
- EP-A- 0 470 310
- WO-A-96/26201
- WO-A-99/02512
- A. P. SHARMA ET AL.: "Structure-Activity-Relationship of Antiestrogens. Effect of the Side Chain and Its Position on the Activity of 2,3-Diaryl-2H-1-benzopyrans" J. MED. CHEM., Bd. 33, 1990, Seiten 3216-3222, XP002170868 in der Anmeldung erwähnt

## Beschreibung

Die vorliegende Erfindung betrifft die 4-Triffuormethyl-2H-benzopyrane der allgemeinen Formel I 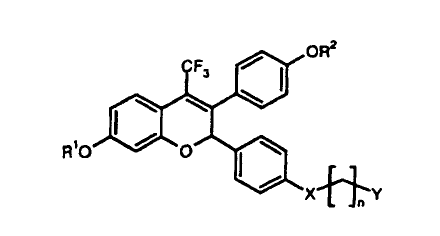 worin
- R¹ und R²: unabhängig voneinander ein Wasserstoffatom, eine gerad- oder verzweigtkettige Alkylgruppe mit bis zu 5 Kohlenstoffatomen, eine gerad- oder verzweigtkettige Alkanoylgruppe mit bis zu 5 Kohlenstoffatomen oder eine Benzoylgruppe,
- X: ein Sauerstoff- oder Schwefelatom oder eine Methylengruppe
- n: 2, 3 oder 4,
- Y: eine Gruppe -NR³R⁴, wobei
R³ und R⁴ unabhängig voneinander ein Wasserstoffatom, ein gerad- oder verzweigtkettiger, gegebenenfalls teilweise fluorierter Alkyl-, Alkenyl- oder Alkinylrest mit bis zu 10 Kohlenstoffatomen, der durch ein bis drei Heteroatome -O- und -S- und Gruppierungen -NR⁵-, worin R⁵ ein Wasserstoffatom oder eine gerad- oder verzweigtkettige Alkylgruppe mit bis zu 5 Kohlenstoffatomen ist, unterbrochen sein kann, ein gegebenenfalls ein- oder zweifach substituierter Aryl- oder Heteroarylrest, ein gegebenenfalls ein- oder zweifach substituierter C₃-C₁₀-Cycloalkylrest, ein gegebenenfalls ein- oder zweifach substituierter C₄-C₁₅-Cycloalkylalkylrest, ein gegebenenfalls ein- oder zweifach substituierter C₇-C₂₀-Aralkylrest, ein gegebenenfalls ein- oder zweifach substituierter Heteroaryl-C₁-C₈-alkylrest oder ein gegebenenfalls substituerter Aminoalkylrest, ein Biphenylenrest oder ein Rest der Formel -C(O)R⁶, worin R⁶ die vorstehend für R³ bzw. R⁴ angegebenen Bedeutungen haben kann, oder aber
- R³ und R⁴: gemeinsam mit dem Stickstoffatom einen 5- bis 7-gliedrigen, gesättigten oder ungesättigten heterocyclischen Ring, der zusätzlich ein Sauerstoff- oder Schwefelatom oder eine Stickstofffunktion =N- bzw.
-NR⁷-, worin R⁷ ein Wasserstoffatom oder eine gerad- oder verzweigtkettige Alkylgruppe mit bis zu 5 Kohlenstoffatomen ist, als Ringglied enthalten kann,
bedeuten,
sowie deren physiologisch verträgliche Additionssalze mit organischen und anorganischen Säuren.

Die vorliegende Erfindung betrifft außer diesen Verbindungen der allgemeinen Formel I, und deren physiologisch verträgliche Additionssalze mit organischen und anorganischen Säuren, diese Verbindungen der allgemeinen Formel I inclusive der Additionssalze enthaltende pharmazeutische Präparate sowie deren Verwendung zur Herstellung von Arzneimitteln.

Zur Bildung von Säureadditionssalzen sind anorganische und organische Säuren geeignet, wie sie dem Fachmann zur Bildung physiologisch verträglicher Salze bekannt sind. Als Additionssalze mit Säuren sind insbesondere die Hydrochloride, Hydrobromide, Acetate, Citrate, Oxalate, Tartrate und die Methansulfonate zu nennen.

Bei einem Alkylrest R¹, R², R⁵ bzw. R⁷ handelt es sich um gerad- oder verzweigtkettige Alkylgruppen mit bis zu 5 Kohlenstoffatomen, wie beispielsweise Methyl, Ethyl, Propyl, Isopropyl, Butyl, Isobutyl, tert.-Butyl, Pentyl, Isopentyl, Neopentyl.

Die als R¹ bzw. R² möglichen Alkanoylgruppen sollen 1 bis 5 Kohlenstoffatome enthalten, wobei Formyl, Acetyl, Propionyl- und Isopropionylgruppen bevorzugt sind.

Als Alkylgruppen R³ und R⁴ sind gerad- oder verzweigtkettige Alkylgruppen mit bis zu 10 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Diese können bis zu 3 Unsättigungen (Doppel- und/oder Dreifachbindungen) aufweisen.

Die Alkylgruppen R³ und R⁴ können teilweise oder vollständig fluoriert oder substituiert sein.
Diese können ebenfalls bis zu 3 Unsättigungen (Doppel- und/oder Dreifachbindungen) aufweisen.

Für den Arylrest R³ bzw. R⁴ und den (Hetero)Arylrest innerhalb des (Hetero)Arylalkylrestes R³ bzw. R⁴ können die folgenden, gegebenenfalls ein- oder mehrfach substituierten Reste stehen:
ein monocyclischer carbocyclischer Rest, beispielsweise der Phenylrest;
ein monocyclischer heterocyclischer Rest, beispielsweise der Thienyl-, Furyl-, Pyrrolyl-, Imidazolyl-, Pyrazolyl-, Pyridyl-, Pyrazinyl-, Pyrimidinyl-, Pyridazinyl-, Thiazolyl-, Oxazolyl-, Furazanyl-, Pyrrolinyl-, Imidazolinyl-, Pyrazolinyl-, Thiazolinyl-, Triazolyl-, Tetrazolylrest, und zwar alle möglichen Isomeren bezüglich der Positionen der Heteroatome;
ein kondensierter carbocyclischer Rest, beispielsweise der Naphthyl- oder Phenanthrenylrest,
ein kondensierter Rest, der sich aus carbocyclischen und heterocyclischen Resten zusammensetzt, beispielsweise der Benzofuranyl-, Benzothienyl-, Benzimidazolyl-, Benzothiazolyl-, Naphto[2,3-b]thienyl-, Thianthrenyl-, Isobenzofuranyl-, Chromenyl-, Xanthenyl-, Phenoxathiinyl-, Indolizinyl-, Isoindolyl-, 3H-Indolyl, Indolyl-, Indazolyl-, Purinyl-, Chinolizinyl-, Isochinolyl-, Chinolyl-, Phthalazinyl-, Naphthyridinyl-, Chinoxalinyl-, Chinazolinyl-, Cinnolinyl-, Pteridinyl-, Carbazolyl-, β-Carbolinyl-, Acridinyl-, Phenazinyl-, Phenothiazinyl-, Phenoxazinyl-, Indolinyl-, Isoindolinyl-, Imidazopyridyl-, Imidazopyrimidinyl- oder
ein kondensiertes polyheterocyclisches System, beispielsweise Furo[2,3-b]pyrrol oder Thieno[2,3-b]furan.

Als Cycloalkylgruppe für die Substituenten R³ und R⁴ kommen substituierte und unsubstituierte Reste mit 3 bis 10 Kohlenstoffatomen in Betracht; vor allem ist die Cyclopropyl- und Cyclopentylgruppe und als Alkylcycloalkylgruppe die Methylcyclopropyl- und Methylcyclopentylgruppe zu nennen.

Die C₇-C₂₀-Aralkylreste in R³ und R⁴ können im Ring bis 14 C-Atome, bevorzugt 6 bis 10 und in der Alkylkette 1 bis 8, bevorzugt 1 bis 4 C-Atome, enthalten.
Ein Heteroaryl-C₁-C₈-alkylrest in R³ und R⁴ weist als Heteroarylteil einen der bereits genannten Heteroarylreste auf; die Alkylkette verfügt über 1 bis 8, bevorzugt über 1 bis 4 C-Atome.
Als Aralkylreste kommen beispielweise in Betracht Benzyl, Phenylethyl, Phenylpropyl, Naphtylmethyl, Naphtylethyl und als Heteroaryl-alkylrest Furylmethyl, Thienylethyl, Pyridylpropyl.
Die Ringe können einfach oder zweifach substituiert sein.

Wenn R³ und R⁴ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält, ist dies insbesondere ein Pyrrolidin-, Piperidin-, Morpholin- oder Piperazinring.
Folgende Reste sind als Substituent Y in erster Linie zu erwähnen:
Piperidin-, Pyrrolidin-, Hexamethylenimino-, Pyrrolidin-, Dimethylamino-, Diethylamino-, Dipropylamino-, N-Methyl-N-phenethylamino-, N-Methyl-N-(3-phenylpropyl)amino-, N-Butyl-N-ethylamino-, Thiomorpholino-, Morpholino-, N-Methyl-N-pentylamino-, N-Isobutyl-N-methylamino-, N-Benzyl-N-methylamino-, N-Butyl-N-methylaminogruppe.

Der fluorierte Alkylrest Z ist ein mindestens einfach fluorierter geradkettiger Alkylrest mit 1 bis 5 oder ein mindestens einfach fluorierter verzweigter Alkylrest mit 3, 4 oder 5 Kohlenstoffatomen.
Als Vertreter für Z seien der Monofluormethyl-, Difluormethyl-, Trifluormethyl-, Pentafluorethyl-, 2,2,2-Trifluorethyl-, *n*- und *i*-Heptafluorpropyl-, 4,4,4-Trifluorbutyl-, 3,3,4,4,4-Pentafluorbutyl-, 2,2,2-Trifluorethyl-, 4,4,4-Trifluorbutyl-, 3,3,4,4,4-Pentafluorbutyl-, 4,4,5,5,5-Pentafluorpentyl- oder die Nonafluorbutylgruppe genannt.

Als Substituenten an den Resten R³ und R⁴ kommen die nachstehenden Substituenten in Betracht, wobei die Reste einfach oder zweifach, identisch oder unterschiedlich, mit diesen Substituenten substituiert sein können:
Halogenatome: Fluor, Chlor, Brom, lod;
Amino-, Mono(C₁₋₈-alkyl)- oder Di(C₁₋₈-alkyl)amino, wobei beide Alkylgruppen identisch oder verschieden sind, insbesondere Methylamino oder Ethylamino, Dimethylamino, Diethylamino oder Methylethylamino; Di(aralkyl)amino, wobei beide Aralkylgruppen identisch oder verschieden sind;
Hydroxylgruppen;
freie, veresterte oder in Form eines Salzes vorliegende Carboxylgruppen: verestert mit einer Carboxycarbonylgruppe, beispielsweise Methoxycarbonyl oder Ethoxycarbonyl; als Salz beispielsweise in Form des Natrium- oder Kaliumsalzes;
Alkylgruppen mit 1 bis 8 Kohlenstoffatomen, wie beispielsweise die Methyl-, Ethyl-, n-oder iso-Propyl-, n-, iso- oder tert.-Butylgruppe, gegebenenfalls substituiert mit einem oder mehreren Halogenatomen, zum Beispiel mit Fluor wie die Trifluormethyl- oder Pentafluorethylgruppe;
Oxo-, Azido-, Cyano-, Nitro- oder Formylgruppen;
Acylgruppen wie Acetyl, Propionyl, Butyryl, Benzoyl;
Acyloxygruppen wie Acetoxy, Reste der Formel-O-CO-(CH₂)ₙ-COOH mit n = 1 bis 5;
C₁-C₄-Alkoxygruppen wie zum Beispiel Methoxy, Ethoxy, Propoxy, Isopropoxy, Butoxy;
Alkylthiogruppen, beispielsweise Methyhhio, Ethylthio, Propylthio, Isopropylthio, Butylthio, alle gegebenenfalls fluoriert;
Carbamoylgruppen;
Alkenylgruppen, beispielsweise Vinyl, Propenyl;
Alkinylgruppen, beispielsweise Ethinyl, Propinyl;
C₆-C₁₂-Arylgruppen wie Phenyl, Furyl, Thienyl, die wiederum ein- bis dreifach substituiert sein können.

Als Substituenten für den Aryl-, Heteroaryl-, Aralkyl- und Heteroarylalkylrest seien insbesondere ein Trifluormethyl-, Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind, oder der 1-Methoxyacetylaminorest genannt.

Freie Hydroxygruppen in den Verbindungen der allgemeinen Formel I können funktionell abgewandelt sein, beispielsweise durch Veretherung oder Veresterung. Als Ether- und Acylreste kommen die dem Fachmann bekannten Reste wie z.B. der Methoxymethyl-, Methoxyethyl-, Ethoxyethyl-, Tetrahydropyranyl-, Tetrahydrofuranyl, Trimethylsilyl-, Triethylsilyl-, tert.-Butyldimethylsilyl-, tert.-Butyldiphenylsilyl-, Tribenzylsilyl-, Triisopropylsilyl-, Methyl-, tert.-Butyl-, Benzyl-, para-Nitrobenzyl-, para-Methoxybenzyl, Formyl-, Acetyl-, Propionyl-, Isopropionyl-, Butyryl-, Pivalyl-, BenzoylRest in Frage. Eine Übersicht befindet sich z.B. in "Protective Groups in Organic Synthesis" Theodora W. Green, John Wiley and Sons).

Eine Variante der Erfindung sieht vor, daß R¹ und R² beide für Wasserstoff stehen.

Gemäß einer anderen Variante stehen R¹ und R² je für eine Methylgruppe.

In einer weiteren Variante sind R¹ und R² je eine Pivaloylgruppe.

Bei dem Glied X handelt es sich vorzugsweise um ein Sauerstoff- oder Schwefelatom.

Der Index n kann gleichermaßen den Wert 2, 3 oder 4 annehmen.

Die Verbindungen der allgemeinen Formel I weisen am Kohlenstoffatom 2 des 2H-Benzopyran-Grundgerüstes ein Asymmetriezentum auf.

Die Erfindung betrifft sowohl die racemischen Gemische der Verbindungen der allgemeinen Formel I als auch die getrennten optischen Isomeren sowie die nichtracemischen Gemische der optischen Isomeren.
Die bei der erfindungsgemäßen Synthese der Verbindungen der allgemeinen Formel I anfallenden Racemate lassen sich nach dem Fachmann bekannten Methoden, beispielsweise mittels HPLC an einer chiralen stationären Phase, trennen. Diese Trennung in die optischen Antipoden kann auf der Stufe der Endverbindung der allgemeinen Formel I, wie dies im Beispiel 1 beschrieben ist, oder auch auf einer früheren racemischen Stufe erfolgen.

Eine Derivatisierung bzw. Funktionalisierung der freien Dihydroxyverbindung zu den Estern (R¹ und/oder R² = Alkanoyl oder Benzoyl) oder Ethern (R¹ und/oder R² = Alkyl) kann an den racemischen Gemischen oder an den getrennten Isomeren erfolgen.
Funktionelle Gruppen R¹ und/oder R², insbesondere für den Fall daß die beiden Substituenten verschieden sein sollen, können auch bereits beim Aufbau der Zwischenstufe des 2,3-Dihydro-4H-1-benzopyran-4-ons eingebracht werden.

Die vorliegende Erfindung betrifft insbesondere die Verbindungen
(+)-7-Hydroxy-3-(4'-hydroxyphenyl)-2-[4"-(2"'-piperidin-1-ylethoxy)-phenyl]-4-(trifluormethyl)-2H-1-benzopyran
(-)-7-Hydroxy-3-(4'-hydroxyphenyl)-2-[4"-(2"'-piperidin-1-ylethoxy)-phenyl]-4-(trifluormethyl)-2H-1-benzopyran
(+)-7-Pivaloyloxy-3-(4'-pivaloyloxyphenyl)-4-trifluormethyl-2-(4"-(2"'-piperidinoethoxy)phenyl)-2H-benzopyran
(-)-7-Pivaloyloxy-3-(4'-pivaloyloxyphenyl)-4-trifluormethyl-2-(4"-(2"'-piperidinoethoxy)-phenyl)-2H-benzopyran
2-[4"-(2"'-Dimethylaminoethoxy)-phenyl]-7-hydroxy-3-(4'-hydroxyphenyl)-4-(trifluormethyl)-2H-1-benzopyran
2-[4"-(2"'-Diethylaminoethoxy)-phenyl]-7-hydroxy-3-(4'-hydroxyphenyl)-4-(trifluormethyl)-2H-1-benzopyran
7-Hydroxy-3-(4'-hydroxyphenyl)-2-[4"-(2"'-N-methyl-N-phenethylaminoethoxy)-phenyl]-4-(trifluormethyl)-2H-1-benzopyran
7-Hydroxy-3-(4'-hydroxyphenyl)-2-{4"-[2"'-N-methyl-N-(3""phenylpropyl)aminoethoxy]-phenyl}-4-(trifluormethyl)-2H-1-benzopyran
2-[4"-(2"'-N-Butyl-N-ethylaminoethoxy)-phenyl]-7-hydroxy-3-(4'-hydroxyphenyl)-4-(trifluormethyl)-2H-1-benzopyran
7-Hydroxy-3-(4'-hydroxyphenyl)-2-[4"-(thiomorpholin-1-ylethoxy)-phenyl]-4-(trifluormethyl)-2H-1-benzopyran
7-Hydroxy-3-(4'-hydroxyphenyl)-2-[4"-(2"'-pyrrolidin-1-ylethoxy)-phenyl]-4-(trifluormethyl)-2H-1-benzopyran
7-Hydroxy-3-(4'-hydroxyphenyl)-2-[4"-(2"'-morpholin-1-ylethoxy)-phenyl]-4-(trifluormethyl)-2H-1-benzopyran
7-Hydroxy-3-(4'-hydroxyphenyl)-2-[4"-(2"'-N-methyl-N-pentylaminoethoxy)-phenyl]-4-(trifluormethyl)-2H-1-benzopyran
7-Hydroxy-3-(4'-hydroxyphenyl)-2-[4"-(2"'-N-isobutyl-N-methylaminoethoxy)-phenyl]-4-(trifluormethyl)-2H-1-benzopyran
2-[4"-(2"'-N-Benzyl-N-methylaminoethoxy)-phenyl]-7-hydroxy-3-(4'-hydroxyphenyl)-4-(trifluormethyl)-2H-1-benzopyran
2-[4"-(2"'-N,N-Di-n-propylaminoethoxy)-phenyl]-7-hydroxy-3-(4'-hydroxyphenyl)-4-(trifluormethyl)-2H-1-benzopyran
2-[4"-(2"'-Hexamethylenimin-1-ylethoxy)-phenyl]-7-hydroxy-3-(4'-hydroxyphenyl)-4-(trifluormethyl)-2H-1-benzopyran
2-[4"-(2"'-N-Butyl-N-methylaminoethoxy)-phenyl]-7-hydroxy-3-(4'-hydroxyphenyl)-4-(trifluormethyl)-2H-1-benzopyran
7-Hydroxy-3-(4'-hydroxyphenyl)-2-[4"-(3"'-piperidin-1-ylpropoxy)-phenyl]-4-(trifluormethyl)-2H-1-benzopyran
2-[4"-(3"'-N-Dimethylaminopropoxy)-phenyl]-7-hydroxy-3-(4'-hydroxyphenyl)-4-(trifluormethyl)-2H-1-benzopyran
2-[4"-(3"'-N-Diethylaminopropoxy)-phenyl]-7-hydroxy-3-(4'-hydroxyphenyl)-4-(trifluormethyl)-2H-1-benzopyran
7-Hydroxy-3-(4'-hydroxyphenyl)-2-[4"-(3"'-N-methyl-N-phenethylaminopropoxy)-phenyl]-4-(trifluormethyl)-2H-1-benzopyran
7-Hydroxy-3-(4'-hydroxyphenyl)-2-{4"-[3"'-N-methyl-N-(3""phenylpropyl)aminopropoxy]-phenyl}-4-(trifluormethyl)-2H-1-benzopyran
2-[4"-(3"'-N-Butyl-N-ethylaminopropoxy)-phenyl]-7-hydroxy-3-(4'-hydroxyphenyl)-4-(trifluormethyl)-2H-1-benzopyran
7-Hydroxy-3-(4'-hydroxyphenyl)-2-[4"-(3"'-thiomorpholin-1-ylpropoxy)-phenyl]-4-(trifluormethyl)-2H-1-benzopyran
7-Hydroxy-3-(4'-hydroxyphenyl)-2-[4"-(3"'-pyrrolidin-1-ylpropoxy)-phenyl]-4-(trifluormethyl)-2H-1-benzopyran
7-Hydroxy-3-(4'-hydroxyphenyl)-2-[4"-(3"'-morpholin-1-ylpropoxy)-phenyl]-4-(trifluormethyl)-2H-1-benzopyran
7-Hydroxy-3-(4'-hydroxyphenyl)-2-[4"-(3"'-N-methyl-N-pentylaminopropoxy)-phenyl]-4-(trifluormethyl)-2H-1-benzopyran
7-Hydroxy-3-(4'-hydroxyphenyl)-2-[4"-(3"'-N-isobutyl-N-methylaminopropoxy)-phenyl]-4-(trifluormethyl)-2H-1-benzopyran 2-[4"-(3"'-N-Benzyl-N-methylaminopropoxy)-phenyl]-7-hydroxy-3-(4'-hydroxyphenyl)-4-(trifluormethyl)-2H-1-benzopyran
2-[4"-(3"'-N,N-Di-n-propylaminopropoxy)-phenyl]-7-hydroxy-3-(4'-hydroxyphenyl)-4-(trifluormethyl)-2H-1-benzopyran
2-[4"-(3"'-Hexamethylenimin-1-ylpropoxy)-phenyl]-7-hydroxy-3-(4'-hydroxyphenyl)-4-(trifluormethyl)-2H-1-benzopyran
2-[4"-(3"'-N-Butyl-N-methylaminopropoxy)-phenyl]-7-hydroxy-3-(4'-hydroxyphenyl)-4-(trifluormethyl)-2H-1-benzopyran
7-Hydroxy-3-(4'-hydroxyphenyl)-2-[4"-(4"'-piperidin-1-ylbutoxy)-phenyl]-4-(trifluormethyl)-2H-1-benzopyran
2-[4"-(4"'-N-Dimethylaminobutoxy)-phenyl]-7-hydroxy-3-(4'-hydroxyphenyl)-4-(trifluormethyl)-2H-1-benzopyran
2-[4"-(4"'-N-Diethylaminobutoxy)-phenyl]-7-hydroxy-3-(4'-hydroxyphenyl)-4-(trifluormethyl)-2H-1-benzopyran
7-Hydroxy-3-(4'-hydroxyphenyl)-2-[4"-(4"'-N-methyl-N-phenethylaminobutoxy)-phenyl]-4-(trifluormethyl)-2H-1-benzopyran
7-Hydroxy-3-(4'-hydroxyphenyl)-2-{4"-[4"'-N-methyl-N-(3""phenylpropyl)aminobutoxy]-phenyl}-4-(trifluormethyl)-2H-1-benzopyran 2-[4"-(4"'-N-Butyl-N-ethylaminobutoxy)-phenyl]-7-hydroxy-3-(4'-hydroxyphenyl)-4-(trifluormethyl)-2H-1-benzopyran
7-Hydroxy-3-(4'-hydroxyphenyl)-2-[4"-(4"'-thiomorpholin-1-ylbutoxy)-phenyl]-4-(trifluormethyl)-2H-1-benzopyran
7-Hydroxy-3-(4'-hydroxyphenyl)-2-[4"-(4"'-pyrrolidin-1-ylbutoxy)-phenyl]-4-(trifluormethyl)-2H-1-benzopyran
7-Hydroxy-3-(4'-hydroxyphenyl)-2-[4"-(4"'-morpholin-1-ylbutoxy)-phenyl]-4-(trifluormethyl)-2H-1-benzopyran
7-Hydroxy-3-(4'-hydroxyphenyl)-2-[4"-(4"'-N-methyl-N-pentylaminobutoxy)-phenyl]-4-(trifluormethyl)-2H-1-benzopyran
7-Hydroxy-3-(4'-hydroxyphenyl)-2-[4"-(4"'-N-isobutyl-N-methylaminobutoxy)-phenyl]-4-(trifluormethyl)-2H-1-benzopyran
2-[4"-(4"'-N-Benzyl-N-methylaminobutoxy)-phenyl]-7-hydroxy-3-(4'-hydroxyphenyl)-4-(trifluormethyl)-2H-1-benzopyran
2-[4"-(4"'-N,N-Di-n-propylaminobutoxy)-phenyl]-7-hydroxy-3-(4'-hydroxyphenyl)-4-(trifluormethyl)-2H-1-benzopyran
2-[4"-(4"'-Hexamethylenimin-1-ylbutoxy)-phenyl]-7-hydroxy-3-(4'-hydroxyphenyl)-4-(trifluormethyl)-2H-1-benzopyran
2-[4"-(4"'-N-Butyl-N-methylaminobutoxy)-phenyl]-7-hydroxy-3-(4'-hydroxyphenyl)-4-(trifluormethyl)-2H-1-benzopyran
(+)-7-Methoxy-3-(4'-methoxyphenyl)-2-[4"-(2"'-piperidin-1-ylethoxy)-phenyl]-4-(trifluormethyl)-2H-1-benzopyran
(-)-7-Methoxy-3-(4'-methoxyphenyl)-2-[4"-(2"'-piperidin-1-ylethoxy)-phenyl]-4-(trifluormethyl)-2H-1-benzopyran
7-Hydroxy-3-(4'-hydroxyphenyl)-2-[4"-(3"'-piperidin-1-yl-1-thiapropyl)-phenyl]-4-(trifluormethyl)-2H-1-benzopyran.

Verbindungen mit antiestrogenen Eigenschaften, d.h. Stoffe mit Hemmwirkungen gegenüber Estrogenen, sind bereits zahlreich beschrieben worden.
Als den vorliegenden Verbindungen der allgemeinen Formel I strukturell am nächsten kommende Verbindungen sind die aus der WO 93/10741 und insbesondere die aus der WO 96/26201 hervorgehenden Verbindungen anzusehen. Die bekannten Verbindungen sind als Estrogenantagonisten und/oder Verbindungen, die Estrogenbiosynthese unterdrücken, beschrieben. Diese bekannten Verbindungen weisen wie die erfindungsgemäßen Verbindungen der allgemeinen Formel I den Grundkörper des 2H-Benzopyrans auf. Die bekannten Verbindungen können in der Position 4 des Grundgerüsts aber keine Perfluoralkylgruppe tragen.
Des weiteren sind noch die in J. Med. Chem. 1990, 33, 3210-3216 und 3216-3222 sowie 3222-3229 beschriebenen 2,3-Diaryl-2H-1-benzopyrane, die in Position 4 lediglich ein Wasserstoffatom als möglichen Substituenten aufweisen können, als Stand der Technik heranzuziehen.
Sharma et al. beschreiben in J. Med. Chem. 1990, 33, 3216-3222 eine Reihe von 2,3-Diaryl-2*H*-1-benzopyranen und untersuchen den Effekt des tert-aminoethoxysubstituierten 2-Arylrestes bzw. des 4-Alkylsubstituenten auf die antiestrogene/estrogene Eigenschaft der Modellverbindungen.

Die Aufgabe der vorliegenden Erfindung bestand darin, Antiestrogene mit gewebeselektiver estrogener Wirkung bereitzustellen, die ein besser ausbalanciertes Wirkprofil hinsichtlich antiestrogener und estrogener Wirkung aufweisen, als die bereits bekannten Verbindungen.
Die Aufgabe wurde durch die erfindungsgemäßen 4-Trifluormethyl-2H-benzopyrane der allgemeinen Formel I gelöst.

Bei den erfindungsgemäßen Verbindungen handelt es um sogenannte Partialagonisten, d. h. um Antiestrogene mit estrogener Partialwirkung wie das Tamoxifen, das Raloxifen oder EM 800.

Im Gegensatz zum Tamoxifen ist bei den Partialagonisten der allgemeinen Formel I die Gewebeselektivität ihrer agonistischen, estrogenen Wirkung stärker ausgeprägt. Insbesondere tritt die agonistische Wirkung am Knochen (knochenprotektive Wirkung), im Herz-Kreislaufsystem und im ZNS (Zentrales Nervensystem) auf, während am Uterus und an der Mamma keine oder nur geringe agonistische Wirkung auftritt. Verbindungen mit einem derartigen Profil werden als Selective Estrogen Receptor Modulators (SERMs) bezeichnet (Structure-Activity Relationships of Selective Estrogen Receptor Modulators: Modifications to the 2-Arylbenzothiophene Core of Raloxifene, T. A. Grese et al, J. Med. Chem. 1997, 40, 146 - 167). Prominentester Vertreter dieser Verbindungsklasse ist das Raloxifen, welches inzwischen als Medikament für die Prävention und die Behandlung der postmenopausalen Osteoporose zugelassen ist.

Das erst kürzlich als selektives Estrogen bekannt gewordene EM 800 [(+)-7-Pivaloyloxy-3-(4'pivaloyloxyphenyl)-4-methyl-2-(4"-(2"piperidinoethoxy)phenyl)-2Hbenzopyran] {J. Steroid Biochem. & Mol. Biol., 69, S. 51 - 84, 1999} war ursprünglich in der WO 96/26201 als reines Antiestrogen beschrieben worden. Bei diesem Bispivalat handelt es sich um ein Prodrug der freien Dihydroxyverbindung.
Im Gegensatz zum EM 800 ist bei Partialagonisten der allgemeinen Formel I die gewebeselektive estrogene Wirkung im allgemeinen stärker ausgeprägt, wobei aber die antagonistische Wirkung mindestens vergleichbar oder besser ist, um den protektiven Effekt des Antiestrogens auf die Mamma und den Uterus ausüben zu können. Mithin handelt es sich bei den gewebeselektiven Estrogenen der vorliegenden Erfindung um Verbindungen mit einem besser ausbalancierten Wirkprofil hinsichtlich estrogener und antiestrogener Wirkung.

Im übrigen zeichnen sich die Verbindungen der allgemeinen Formel I gemäß vorliegender Anmeldung im Vergleich zu dem bereits bekannten EM 800 durch eine höhere Luftstabilität aus. Die neuen Verbindungen lassen sich daher einfacher als pharmazeutische Präparate formulieren. Dieser Umstand wirkt sich auch vorteilhaft auf die Lagerstabilität der fertig formulierten pharmazeutischen Präparate aus. Eine vergleichbare Lagerstabilität der erfindungsgemäßen pharmazeutischen Präparate im Vergleich zu beispielsweise EM 800 enthaltenden Präparaten läßt sich mit einer weniger aufwändigen Formulierungstechnik und galenischen Formulierungsform erreichen.

### Pharmakologische Untersuchung der erfindungsgemäßen Verbindungen

Der Einfluß der erfindungsgemäßen Verbindungen auf den Uterus wurde im Uteruswachstumstest (estrogene Wirkung) und im Antiuteruswachstumstest (antiestrogene Wirkung), beide an der infantilen Ratte durchgeführt, untersucht.

### Estrogene/antiestrogene Wirkung in vivo

### Uteruswachstumstest an der infantilen Ratte (n=5 Tiere/Gruppe)

Sowohl Uterus wie auch Vagina zeigen bei infantilen Tieren bei deren Behandlung mit einer estrogen wirksamen Substanz eine von der estrogenen Wirksamkeit abhängige Gewichtszunahme. Am Uterus kommt es unter estrogener Wirkung zudem zu einer Proliferation und Höhenzunahme des luminalen Epithels. Immature, intakte Ratten (Körpergewicht 40-50g) erhalten über 3 Tage (d1-d3) die Substanz s.c.. Am Tag 4 (d4) werden die Tiere mit CO₂ getötet. Die Uteri werden herauspräpariert und gewogen. Ein Uterusstück, vorzugsweise ein Uterushom, wird für die histologische Auswertung in Formaldehyd fixiert und in Paraffin eingebettet. Die Stimulierung der Organgewichte (bezogen auf mg/100g Körpergewicht) sowie die Epithelhöhe wird in prozentualer Stimulierung im Vergleich zur Referenzverbindung 17β-Estradiol angegeben. (Substitutiondosis E₂ 0,3 µg/Tier).

Die erfindungsgemäßen Verbindungen weisen keine oder nur geringe stimulierende Wirkung auf den Uterus auf.

### Antiuteruswachstumstest an der infantilen Ratte (n=5 Tiere/Gruppe)

Der Uterus infantiler estrogensubstituierter Ratten kann als Testmodell genutzt werden, um eine direkte Wirkung von Substanzen mit antiestrogenen Eigenschaften nachzuweisen. Der Parameter der Estrogenwirkung ist das bei infantilen Ratten durch Estradiol induzierte Uteruswachstum, das durch gleichzeitige Gabe einer Substanz mit antiöstogener Wirkung gehemmt wird.

Die Testsubstanzen werden s.c. an 3 aufeinanderfolgenden Tagen (d1-d3) in Kombination mit einer Substitutionsdosis 0.3 µg/Tier/Tag 17β-Estradiol behandelt.
Als Positivkontrolle dient 17β-Estradiol alleine, als Negativkontrolle die Vehikel-Gruppe. An Tag 4 (d4) werden die Tiere getötet, Uteri und Vaginae werden herauspräpariert und gewogen. Die Organgewichte werden auf mg/100 g Körpergewicht umgerechnet, dann der Mittelwert und die Standardabweichung für jede Dosierung berechnet. Die Hemmung des durch 17β-Estradiol induzierten Uterus- bzw. Vaginalwachstums wird als Hemmung in % angegeben.
Die erfindungsgemäßen Verbindungen weisen zum großen Teil eine deutlich ausgeprägte Hemmung des durch 17β-Estradiol induzierten Uteruswachstums auf.

Die erfindungsgemäßen Verbindungen sind somit hinsichtlich ihrer Wirkung am Uterus den Verbindungen des Standes der Technik im Sinne vorliegender Erfindung dahingehend überlegen, daß sie an diesem Organ geringere oder gar keine estrogene Wirkung aufweisen.

### Knochenuntersuchungen

### Methode

3 Monate alte weibliche Ratten werden ovarektomiert und unmittelbar nach der Operation 28 Tage lang 1 mal täglich mit der Testverbindung behandelt. Die Applikation erfolgt subcutan in Arachisöl/Ethanol oder peroral. Die Tiere werden am Tag nach der letzten Applikation getötet und Tibia sowie die Uteri entnommen. Die Uteri werden gewogen, fixiert und für histologische Untersuchungen aufgearbeitet. Die Bestimmung der Knochendichte erfolgt ex vivo an präparierten Langknochen mittels pQCT (Quantitative Computertomographie). Die Messungen werden im Abstand von 4 - 6 mm vom Gelenkkopf der proximalen Tibia durchgeführt.

Durch die Ovarektomie vermindert sich die Dichte des trabekulären Knochens im gemessenen Bereich von ca. 400 mg Ca²⁺/cm³ auf ca. 300 mg Ca²⁺/cm³. Durch die Behandlung mit einer Verbindung der allgemeinen Formel I gemäß vorliegender Erfindung (Dosierung von 0.1 mg/kg/Tier/Tag p.o.) wird der Abbau der Knochendichte in der proximalen Tibia verhindert bzw. gehemmt. (+)-7-Hydroxy-3-(4'-hydroxyphenyl)-2-[4"-(2"'-piperidin-1-ylethoxy)-phenyl]-4-(trifluormethyl)-2H-1-benzopyran bewirkt in diesem Test eine Knochenprotektion im Vergleich zur scheinoperierten Kontrolle [sham] von 73%.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die nachstehenden Indikationen, nämlich zur Behandlung von estrogenabhängigen Krankheiten und Tumoren und von Arzneimitteln für die Hormonsubstitutions-Therapie (HRT) sowie zur Prävention und Behandlung der Osteoporose.

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u. ff.; H.v. Czetsch-Lindenwald, Hilfsstoffe für Pharmazie und angrenzende Gebiete; Pharm. Ind. Heft 2, 1961, Seite 72 u. ff.; Dr. H.P. Fiedier, Lexikon der Hilfsstoffe für Pharmazie, Kosmetik und angrenzende Gebiete Cantor KG. Aulendorf in Württemberg 1971.

Die Verbindungen können oral oder parenteral, beispielsweise intraperitoneal, intramuskulär, subkutan, perkutan, nasal oder pulmonal verabreicht werden. Die Verbindungen können auch in das Gewebe implantiert werden.

Die zu verabreichende Menge der Verbindungen schwankt innerhalb eines weiten Bereichs und kann jede wirksame Menge abdecken. In Abhängigkeit des zu behandelnden Zustandes und der Art der Verabreichung kann die Menge der verabreichten Verbindung 0,001 bis 25 mg/kg Körpergewicht, vorzugsweise 0,01 bis 5 mg/kg Körpergewicht, je Tag betragen.
Beim Menschen entspricht dies einer täglichen Dosis von 0,05 bis 1250 mg.
Die bevorzugte tägliche Dosierung beim Menschen ist 0,5 bis 250 mg. Dies gilt insbesondere für die Tumortherapie.
Eine Dosierungseinheit enthält erfindungsgemäß 0,025 bis 250 mg einer oder mehrerer Verbindungen der allgemeinen Formel I.

Zur oralen Verabreichung kommen Kapseln, Pillen, Tabletten, Dragèes usw. infrage. die Dosierungseinheiten können neben dem Wirkstoff einen pharmazeutisch verträglichen Träger, wie zum Beispiel Stärke, Zucker, Sorbit, Gelantine, Gleitmittel, Kieselsäure, Talkum usw., enthalten. Die einzelnen Dosierungseinheiten für die orale Applikation können beispielsweise 5 bis 500 mg des Wirkstoffs enthalten.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656).

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen der allgemeinen Formel I können auch in Form einer Lösung formuliert werden, die für die orale Verabreichung bestimmt ist und die neben der aktiven Verbindung der allgemeinen Formel I enthält
a) ein pharmazeutisch verträgliches Öl und/oder
b) eine pharmazeutisch verträgliche lipophile oberflächenaktive Substanz und/oder
c) eine pharmazeutisch verträgliche hydrophile oberfächenaktive Substanz und/oder
d) ein pharmazeutisch verträgliches wassermischbares Lösungsmittel.
Hierzu wird außerdem auf die WO 97/21440 verwiesen.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder Intrauterinsystemen (z.B. Pessare, Spiralen, IUSs, MIRENA®) für die lokale Verabreichung eignen sich verschiedene Polymere wie zum Beispiel Silikonpolymere, Ethylenvinylacetat, Polyethylen oder Polypropylen.

Erfindungsgemäß können die Verbindungen der allgemeinen Formel I auch mit Liposomen verkapselt werden.

Die erfindungsgemäßen Verbindungen eignen sich zur Therapie von estrogenabhängigen Erkrankungen, zum Beispiel Mammacarcinom (second-line Therapie des Tamoxifen-resistenten Mammacarcinoms; zur adjuvanten Behandlung des Mammacarcinoms anstelle von Tamoxifen), Endometriumcarcinom, Prostatacarcinom, Prostatahyperplasie, anovulatorische Infertilität und Melanom.
Die Verbindungen der allgemeinen Formel I können gemeinsam mit Antigestagenen (kompetitiven Progesteronantagonisten) zur Behandlung hormonabhängiger Tumoren verwendet werden (EP 310 542 A).
Weitere Indikationen, in denen die Verbindungen der allgemeinen Formel zum Einsatz kommen können, ist der männliche Haarausfall, eine diffuse Alopecie, eine durch eine Chemotherapie hervorgerufene Alopecie sowie Hirsutismus (Hye-Sun Oh und Robert C. Smart, Proc. Natl. Acad. Sci. USA, 93 (1996) 12525 - 12530). Außerdem können die Verbindungen der allgemeinen Formel I zur Herstellung von Medikamenten zur Behandlung der Endometriose und von Endometrialkarzinomen verwendet werden.
Ferner kann man die Verbindungen der allgemeinen Formel I zur Herstellung pharmazeutischer Zusammensetzungen für die männliche und weibliche Fertilitätskontrolle einsetzen (männliche Fertilitätskontrolle: DE-A 195 10 862.0).

Die Verbindungen der allgemeinen Formel I können, sowohl nach oraler als auch parenteraler Gabe, für die folgenden Indikationen eingesetzt werden:
Linderung der Symptome der Andropause und Menopause, d.h. zur männlichen und weiblichen Hormonersatz-Therapie (HRT), und zwar sowohl zur Prävention als auch zur Behandlung; zur Behandlung der mit einer Dysmenorrhoe einhergehenden Beschwerden; Behandlung dysfunktioneller uteriner Blutungen; Behandlung der Akne; Prävention und Behandlung kardiovaskulärer Erkrankungen; Behandlung von Hypercholesterinämie und Hypertipidämie; Prävention und Behandlung der Atherosclerose; zur Hemmung der Proliferation der arteriellen Glattmuskelzellen; zur Behandlung des Atemnotsyndroms bei Neugeborenen; Behandlung des primären pulmonaren Bluthochdrucks; zur Vorbeugung und Behandlung der Osteoporose (Black, L.J., Sato,M.,Rowley, E.R.,Magee, D.E., Bekele, A., Williams, D.C., Cullinan, G.J., Bendele, R., Kauffman, R.F., Bensch, W.R., Frolik, C.A., Termine, J.D. and Bryant, H.U.: Raloxifene [LY 139481 HCl] prevents bone loss and reduces serum cholesterol without causing uterine hypertrophy in ovariectomized rats; J. Clin. Invest. 93: 63 - 69, 1994); zur Vorbeugung des Knochenverlusts bei postmenopausalen Frauen, bei hysterektomierten Frauen oder bei Frauen, die mit LHRH Agonisten oder Antagonisten behandelt wurden; Hemmung der Spermienreifung; Behandlung von rheumatoider Arthritis; zur Vorbeugung der Alzheimer'schen Krankheit und anderer neurodegenerativer Erkrankungen; Behandlung der Endometriose; Behandlung von Myomen; Behandlung von Myomen und der Endometriose in Kombination mit LHRH-Analoga; Behandlung hormonabhängiger Tumoren, z.B. des Mammacarcinoms, Behandlung prostatischer Erkrankungen, insbesondere Prävention und Behandlung der Prostatahyperplasie.
Außerdem eignen sich die erfindungsgemäßen Verbindungen aufgrund ihres pharmakologischen Profils sowohl für die männliche als auch für die weibliche Kontrazeption.

Die Verbindungen können auch in Kombination mit dem natürlichen Vitamin D3 oder mit Calcitriol-Analoga oder anderen, den Knochenaufbau fördemden Verbindungen, z. B. Fluoriden, BMP's, Statinen, etc., für den Knochenaufbau oder als unterstützende Therapie zu Therapien, welche einen Knochenmassenverlust verursachen (beispielsweise eine Therapie mit Glucocorticoiden, Chemotherapie) eingesetzt werden.

Schließlich können die Verbindungen der allgemeinen Formel I in Verbindung mit Progesteronrezeptor-Antagonisten oder in Verbindung mit reinen Estrogenen verwendet werden, und zwar insbesondere zur Verwendung in der Hormonersatz-Therapie und zur Behandlung gynäkologischer Störungen und für die weibliche Fertilitätskontrolle.
Ein therapeutisches Produkt, enthaltend ein Estrogen und ein reines Antiestrogen für gleichzeitige, sequentielle oder getrennte Anwendung für die selektive Estrogentherapie perimenopausaler oder postmenopausaler Zustände ist bereits in der EP-A 0 346 014 beschrieben.

Die erfindungsgemäßen Verbindungen der allgemeinen Formel I können wie in den Beispielen beschrieben hergestellt werden. Durch analoge Vorgehensweise unter Verwendung homologer Reagenzien zu den in den Beispielen beschriebenen Reagenzien lassen sich alle Verbindungen der allgemeinen Formel I erhalten.
So lassen sich mit der in den Beispielen 1 und 2 beschriebenen Methode durch analoge Abwandlung auch andere erfindungsgemäße Verbindungen herstellen, wenn im Reaktionsschritt c) anstelle von 1-(2-Chlorethyl)-piperidinhydrochlorid ein entsprechend der gewünschten Endverbindung ausgesuchtes Hydrochlorid eingesetzt wird.

Als Verfahren zum Aufbau der Seitenkette -X-[CH₂]ₙ-Y in den erfindungsgemäßen Verbindungen sind insbesondere auch die in der WO 96/26201 und in der WO 93/10741 beschriebenen Verfahren geeignet.

Soll X in den gewünschten Verbindungen der allgemeinen Formel I für eine Methylengruppe stehen, so wird das im Syntheseschritt c) zunächst als Zwischenprodukt erhaltene 2-(4-Hydroxyphenyl)-7-(tetrahydropyran-2-yloxy)-3-[4-(tetrahydropyran-2-yloxy)-phenyl]-2,3-dihydro-4H-1-benzopyran-4-on in die entsprechende 2-[(4-Trifluormethylsulfonyloxy)-phenyl)-Verbindung überführt. Dieses Triflat wird Palladium(0)-katalysiert mit einer Verbindung HC≡C-(CH₂)ₙ₋₁-Y (Bedeutung von Y siehe allgemeinen Formel I) im Sinne einer Sonogashira-Kupplung [mit Pyrrolidin als Base, Tetrakis(triphenylphosphin)-palladium als Katalysator unter Zusatz von Kupfer (I)halogenid, insbesondere -iodid, bei erhöhter Temperatur (ca. 70°C)] umgesetzt und das entstandene α,β-Alkin anschließend hydriert. Anstelle der Verbindung HC≡C-(CH₂)ₙ₋₁-Y kann die Sonogashira-Kupplung auch mit einer ω-Halogenverbindung HC≡C-(CH₂)ₙ₋₁-Hal (Hal = Cl, Br) durchgeführt und das Halogenid anschließend, vor oder nach der Hydrierung, basenkatalysiert gegen den letztendlich gewünschten Aminrest Z ausgetauscht werden.

Alternativ können die Verbindungen der allgemeinen Formel I, worin X eine Methylengruppe darstellt, über die im nachstehenden Schema gezeigten Reaktionssequenz dargestellt werden:

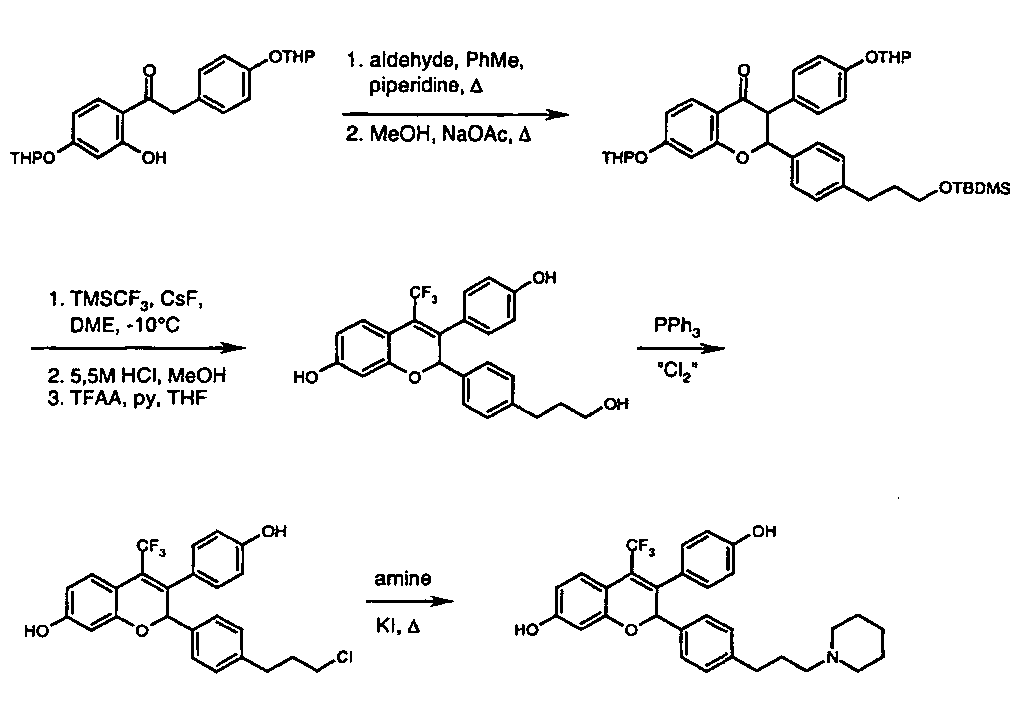

Die Verseifung von Estergruppierungen sowie Veresterung und Veretherung freier Hydroxygruppen erfolgt jeweils nach etablierten Verfahren der organischen Chemie. Durch Beachtung der unterschiedlichen Reaktivität der veresterten und freien 7- und 4'-Hydroxygruppe lassen sich die 7,4'-Diester selektiv spalten und dann gezielt weiter funktionalisieren.
Monoalkylether lassen sich auch durch Auftrennung des entsprechenden Alkylierungsgemisches von den dialkylierten Verbindungen abtrennen.

Die Säureadditionssalze der Verbindungen der allgemeinen Formel I lassen sich ebenfalls nach gängigen Verfahren aus den Verbindungen der allgemeinen Formel I herstellen.

Die nachfolgenden Beispiele dienen der näheren Erläuterung der Erfindung, ohne diese darauf einschränken zu wollen.

### Beispiel 1

### (+)-7-Hydroxy-3-(4-hydroxyphenyl)-2-[4-(2-piperidin-1-ylethoxy)-phenyl]-4-(trifluormethyl)-2H-1-benzopyran

### Beispiel 2

### (-)-7-Hydroxy-3-(4-hydroxyphenyl)-2-[4-(2-piperidin-1-ylethoxy)-phenyl]-4-(trifluormethyl)-2H-1-benzopyran

### a) 1-(2,4-Dihydroxyphenyl)-2-(4-hydroxyphenyl)-ethanon

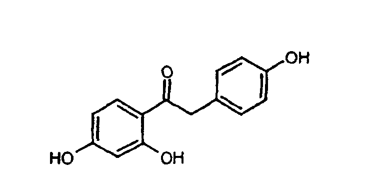

44,6 g (405 mmol) Resorcin und 67,7 g (445 mmol) 4-Hydroxyphenylessigsäure in 120 ml Toluol werden mit 150 ml BF₃^{·}OEt₂ versetzt und 3 Stunden bei 100°C gerührt. Man läßt abkühlen, bei 0-5°C werden 200 ml 12% Natriumacetatlösung hinzugefügt und über Nacht wird bei Raumtemperatur stehengelassen. Die Kristalle werden abgesaugt und zweimal mit 500 ml Wasser und einmal mit 200 ml 12% Natriumacetatlösung gewaschen. Die Kristalle werden erneut mit 600 ml 12% Natriumacetatlösung über Nacht stehengelassen, abgesaugt und mit 300 ml Wasser gewaschen. Anschließend wird aus 400 ml Ethanol und 1500 ml Wasser umkristallisiert. Die Kristalle werden bei 50°C im Vakuum getrocknet. Es fallen 71,8 g (Schmp.: 186°C) an.

### b) 1-[2-Hydroxy-4-(tetrahydropyran-2-yloxy)-phenyl]-2-[4-(tetrahydropyran-2-yloxy)-phenyl]-ethanon

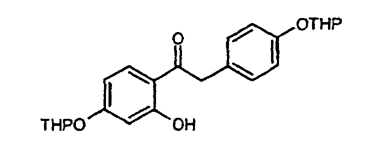

Bei 0°C werden zu 40 g 1-(2,4-Dihydroxyphenyl)-2-(4-hydroxyphenyl)-ethanon in 152 ml Dihydropyran 4,8 mg p-Toluofsulfonsäure zugegeben und 2,5 Stunden gerührt. Es werden 100 ml gesättigte Natriumhydrogencarbonatlösung und 250 ml Essigester zugefügt, die Phasen werden getrennt, und die wäßrige Phase wird einmal mit 250 ml Essigester extrahiert. Die vereinigten organischen Phasen werden einmal mit 80 ml gesättigter Natriumhydrogencarbonatlösung und einmal mit gesättigter Natriumchloridlösung gewaschen, über Magnesiumsulfat getrocknet und am Rotationsverdampfer eingeengt. Der Rückstand wird mit 800 ml Hexan versetzt, 2 Stunden bei Raumtemperatur gerührt und 20 Stunden bei -20°C aufbewahrt. Die Kristalle werden abgesaugt und in 400 ml Hexan resuspendiert und einige Minuten bei Raumtemperatur gerührt. Nach erneutem Absaugen werden 45,3 g Kristalle (Schmp.: 105-106°C) erhalten.

### c) 2-[4-(2-piperidin-1-ylethoxy)-phenyl]-7-(tetrahydropyran-2-yloxy)-3-[4-(tetrahydropyran-2-yloxy)-phenyl]-2,3-dihydro-4H-1-benzopyran-4-on

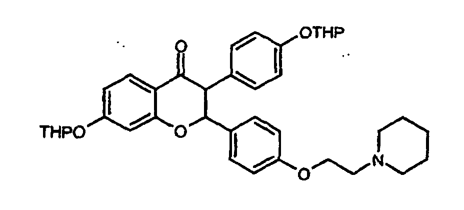

45,3 g 1-[2-Hydroxy-4-(tetrahydropyran-2-yloxy)-phenyl]-2-[4-(tetrahydropyran-2-yloxy)-phenyl]-ethanon, 13,86 g 4-Hydroxybenzaldehyd und 3,55 ml Piperidin in 900 ml Toluol werden 60 Stunden am Wasserabscheider unter einer Stickstoffatmosphäre gerührt. Die abgekühlte Lösung wird am Rotationsverdampfer eingeengt. Der Rückstand wird in 800 ml Aceton und 50 ml Wasser gelöst und mit 24,16 g 1-(2-Chlorethyl)-piperidinhydrochlorid und 85,09 g Cäsiumcarbonat versetzt. Es wird 19 Stunden unter Rückfluß gerührt. Man läßt auf Raumtemperatur abkühlen. Der Niederschlag wird abgesaugt und mit Aceton gewaschen. Die vereinigten Filtrate werden am Rotationsverdampfer eingeengt und über Kieselgel mit Essigester/Methanol gereinigt. Es werden 33 g gewonnen.

### d) 4,7-Dihydroxy-3-(4-hydroxyphenyl)-2-[4-(2-piperidin-1-ylethoxy)-phenyl]-4-(trifluormethyl)-2,3-dihydro-4H-1-benzopyran

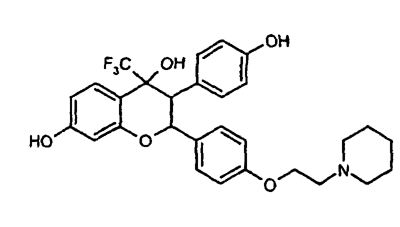

5 g 2-[4-(2-piperidin-1-ylethoxy)-phenyl]-7-(tetrahydropyran-2-yloxy)-3-[4-(tetrahydropyran-2-yloxy)-phenyl]-2,3-dihydro-4H-1-benzopyran-4-on in 50 ml Ethylenglycoldimethylether werden mit 5 ml (Trifluormethyl)-trimethylsilan und 150 mg Cäsiumfluorid versetzt und bei Raumtemperatur gerührt. Nach 48 Stunden werden noch 2 ml (Trifluormethyl)-trimethylsilan und eine Spatelspitze Cäsiumfluorid zugefügt. 24 Stunden später werden die flüchtigen Bestandteile am Rotationsverdampfer abgezogen. Der Rückstand wird in 25 ml Eisessig und 2,5 ml Wasser gelöst und 4 Stunden bei 90°C unter einer Argonatmosphäre gerührt. Nachdem Abkühlen wird am Rotationsverdampfer eingeengt und mit 60 ml Essigester und 30 ml 10% Natriumcarbonatlösung versetzt. Nach 30 Minuten des Nachrührens bei Raumtemperatur werden die Phasen separiert und die wäßrige Phase wird dreimal mit 100 ml Essigester ausgeschüttelt. Die vereinigten organischen Phasen werden zweimal mit 40 ml Wasser gewaschen, über Magnesiumsulfat getrocknet und eingeengt. Das Produkt wird über Kieselgel mit Dichlormethan/Methanol gereinigt. Es werden 1.15 g gewonnen.
MS (EI, 70 eV, 150°C) : m/e = 529 (M⁺, 0,23%), 511 (M-H₂O⁺, 0,08%).

### e) 7-Hydroxy-3-(4-hydroxyphenyl)-2-[4-(2-piperidin-1-ylethoxy)-phenyl]-4-(trifluormethyl)-2H-1-benzopyran

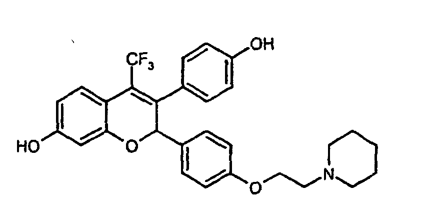

Zu 1,15 g 4,7-Dihydroxy-3-(4-hydroxyphenyl)-2-[4-(2-piperidin-1-ylethoxy)-phenyl]-4-(trifluormethyl)-2,3-dihydro-4H-1-benzopyran in 20 ml THF werden gleichzeitig 0,88 ml Trifluoressigsäureanhydrid und 1,37 ml Pyridin zugetropft. Es wird 4 Stunden bei Raumtemperatur gerührt, bevor 70 ml Essigester und 20 ml 10% Natriumcarbonatlösung zugesetzt werden. Es wird noch 20 Minuten bei Raumtemperatur gerührt. Die Phasen werden getrennt, und die wäßrige Phase wird dreimal mit 40 ml Essigester ausgeschüttelt. Die vereinigten organischen Phasen werden zweimal mit 20 ml Wasser gewaschen, über Magnesiumsulfat getrocknet und eingeengt. Das Produkt wird über Kieselgel mit Dichlormethan/Methanol vorgereinigt und anschließend mit der HPLC (Nucleosil 50-7, Dichlormethan/Methanol 85:15, 40 ml/min) gereinigt. Es werden 590 mg erhalten.
Anschließend werden 825 mg des Racemates mit Hilfe chiraler HPLC (Chiralpak AD 20µ, 250 x 60 mm, Hexan (0,1 % Triethylamin) : Ethanol (0,1% Triethylamin) 9:1, 90 ml/min) getrennt.
Vom (+)-Enantiomer werden 290 mg und vom (-)-Enantiomer 300 mg gewonnen.
(+)-Enantiomer: [α]D = +279,5° (0,502 in Methanol)
(-)-Enantiomer: [α]D = -280° (0,516 in Methanol)

### Beispiel 3

### 2-[4-(2-dimethylaminoethoxy)-phenyl]-7-hydroxy-3-(4-hydroxyphenyl)-4-(trifluormethyl)-2H-1-benzopyran

### a) 2-[4-Hydroxyphenyl]-7-(tetrahydropyran-2-yloxy)-3-[4-(tetrahydropyran-2-yloxy)-phenyl]-2,3-dihydro-4H-1-benzopyran-4-on

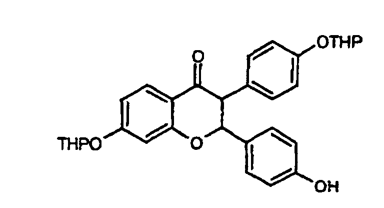

Zu 20 g 1-[2-Hydroxy-4-(tetrahydropyran-2-yloxy)-phenyl]-2-[4-(tetrahydropyran-2-yloxy)-phenyl]-ethanon, 6,51 g 4-Hydroxybenzaldehyd und 1,44 ml Piperidin in 320 ml Toluol werden 5 Stunden bei 80°C Badtemperatur und 30 Stunden am Wasserabscheider unter einer Stickstoffatmosphäre gerührt. Die abgekühlte Lösung wird am Rotationsverdampfer eingeengt. Der Rückstand wird zweimal in Dichlormethan aufgenommen und am Rotationsverdampfer bis zur Trockene eingeengt.

### b) 2-[4-(2-Chlorethoxy)-phenyl]-7-(tetrahydropyran-2-yloxy)-3-[4-(tetrahydropyran-2-yloxy)-phenyl]-2,3-dihydro-4H-1-benzopyran-4-on

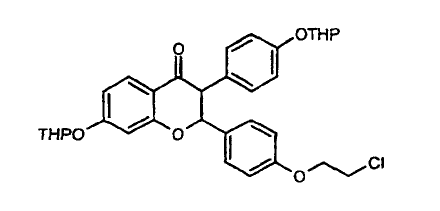

2,19 g 2-[4-Hydroxyphenyl]-7-(tetrahydropyran-2-yloxy)-3-[4-(tetrahydropyran-2-yloxy)-phenyl]-2,3-dihydro-4H-1-benzopyran-4-on, 3,6 g 1-Brom-2-chlorethan und 2,21 g Kaliumcarbonat werden bei 65°C Badtemperatur über Nacht in 20 ml Aceton gerührt. Nach dem Abkühlen wird der Niederschlag abgesaugt und mit tert-Butylmethylether gewaschen. Das Filtrat wird am Rotationsverdampfer bis zur Trockene eingeengt. Der Rückstand wird über Kieselgel mit Hexan/Essigester 9:1 chromatographiert. Es werden 0,838 g Produkt gewonnen.
MS (Cl, NH₃, 70 eV, 150°C) : m/e = 579 (M+H⁺, 36,4%), 495 (77,9%), 410 (42,5%).

### c) 2-[4-(2-Chlorethoxy)-phenyl]-4,7-dihydroxy-3-(4-hydroxyphenyl)-4-(trifluormethyl)-2,3-dibydro-4H-1-benzopyran

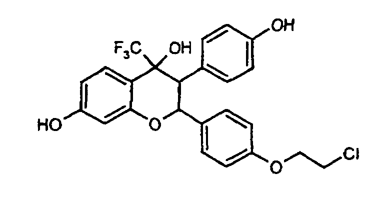

12,7 g 2-[4-(2-Chlorlethoxy)-phenyl]-7-(tetrahydropyran-2-yloxy)-3-[4-(tetrahydropyran-2-yloxy)-phenyl]-2,3-dihydro-4H-1-benzopyran-4-on in 120 ml Ethylenglycoldimethylether werden mit 11,5 ml (Trifluormethyl)-trimethylsilan und 200 mg Cäsiumfluorid versetzt und bei -10°C 2 Stunden gerührt. Anschließend wird die Reaktionslösung in 300 ml gesättigte Natriumchloridlösung gegossen. Es wird dreimal mit 300 ml Essigester ausgeschüttelt. Die vereinigten organischen Phasen werden über Magnesiumsulfat getrocknet und am Rotationsverdampfereingeengt. Das anfallende Rohprodukt wird über Kieselgel mit Hexan und Hexan/Essigester 95:5 gereinigt. Es werden 5,7 g gewonnen.
2,6 g 2-[4-(2-Chlorethoxy)-phenyl]-7-hydroxy-3-(4-hydroxyphenyl)-4-(trifluormethyl)-4-(trimethylsiloxy)-2,3-dihydro-4H-1-benzopyran werden in 70 ml Methanol gelöst und mit 15 ml 5,5 M HCl 4 Stunden bei RT gerührt. Die flüchtigen Bestandteile werden am Rotationsverdampfer abgezogen, der verbleibende Rückstand wird mit 40 ml Wasser versetzt und dreimal mit 50 ml Dichlormethan extrahiert. Die vereinigten organischen Phasen werden zweimal mit 40 ml Wasser gewaschen, über Magnesiumsulfat getrocknet und am Rotationsverdampfer eingeengt. Der Rückstand wird über Kieselgel mit Hexan und Hexan/Essigester 8:2 gereinigt. Es werden 1,5 g Produkt gewonnen.
MS (EI, 70 eV, 150°C) : m/e = 480 (M⁺, 2,3%), 462 (M-H₂O⁺, 0,3%), 411 (1,2%), 393 (1,3%), 307 (2,8%), 274 (100%).

### d) 2-[4-(2-Chlorethoxy)-phenyl]-7-hydroxy-3-(4-hydroxyphenyl)-4-(trifluormethyl)-2H-1-benzopyran

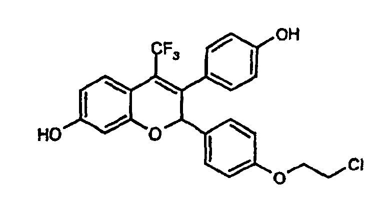

Bei 0-5°C werden zu 2,87 g 2-[4-(2-Chlorethoxy)-phenyl]-4,7-dihydroxy-3-(4-hydroxyphenyl)- 4-(trifluormethyl)-2,3-dihydro-4H-1-benzopyran in 60 ml THF gleichzeitig 2,87ml Trifluoressigsäureanhydrid und 3,84 ml Pyridin zugetropft. Es wird 3 Stunden bei Raumtemperatur nachgerührt, bevor 50 ml gesättigte Natriurnchloridlösung zugesetzt wird. Es wird dreimal mit 50 ml Essigester ausgeschüttelt. Die vereinigten organischen Phasen werden zweimal mit 40 ml Wasser gewaschen, über Magnesiumsulfat getrocknet und am Rotationsverdampfer eingeengt. Der verbleibende Rückstand wird mit Hexan und Hexan/Essigester 8:2 gereinigt, so dass 1,96 g Produkt gewonnen werden.
MS (EI, 70 eV, 150°C) : m/e = 462 (M⁺, 16,5%), 393 (M-CF₃⁺, 63,1 %), 369 (43,3%), 307 (100%).

### e) 2-[4-(2-dimethylaminoethoxy)-phenyl]-7-hydroxy-3-(4-hydroxyphenyl)-4-(trifluormethyl)-2H-1-benzopyran

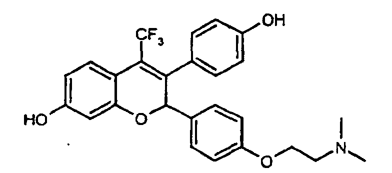

0,035 g 2-[4-(2-Chlorethoxy)-phenyl]-7-hydroxy-3-(4-hydroxyphenyl)-4-(trifluormethyl)-2H-1-benzopyran in 0,5 ml 1-Methyl-2-pyrrolidon werden mit 10,4 mg Kaliumcarbonat, einer Spatelspitze Kaliumiodid und 1 ml Dimethylamin (2M in THF) versetzt und bei RT gerührt. Nach jeweils 24 Stunden des Nachrührens wird zweimal 1 ml Dimethylaminlösung zugefügt. Insgesamt wird 144 Stunden gerührt. Es werden 30 ml Dichlormethan zugefügt, und die organische Phase wird zweimal mit 10 ml Wasser gewaschen, über Magnesiumsulfat getrocknet und am Rotationsverdampfer eingeengt. Präparative Dünnschichtchromatographie mit Dichlormethan/Methanol 9:1 ergibt 15 mg Produkt.

### Beispiele 4 bis 16

Analog werden die anderen Amine (2 Moläquivalente) mit 35 mg 2-[4-(2-Chlorethoxy)-phenyl]-7-hydroxy-3-(4-hydroxyphenyl)-4-(trifluormethyl)-2H-1-benzopyran umgesetzt, wobei die weniger flüchtigen Amine bei 80°C 6 Stunden gerührt werden. Für die chromatographische Reinigung muss nur das Verhältnis von Dichlormethan zu Methanol angepasst werden.

| **Beispiele** | **Amin** | **Ausbeute** |
|---|---|---|
| 4 | N,N-Diethylamin | 8 mg |
| 5 | N-Methyl-N-phenethylamin | 20 mg |
| 6 | N-Methyl-N-(3-phenylpropyl)amin | 23 mg |
| 7 | N-Butyl-N-ethylamin | 6 mg |
| 8 | Thiomorpholin | 17 mg |
| 9 | Pyrrolidin | 11 mg |
| 10 | Morpholin | 18 mg |
| 11 | N-Methyl-N-pentylamin | 12 mg |
| 12 | N-Isobutyl-N-methylamin | 7 mg |
| 13 | N-Benzyl-N-methylamin | 16 mg |
| 14 | N,N-Dipropylamin | 3 mg |
| 15 | Hexamethylenimin | 8 mg |
| 16 | N-Butyl-N-methylamin | 7,5 mg |

### Beispiel 17

### 7-Hydroxy-3-(4-hydroxyphenyl)-2-[4-(3-piperidin-1-ylpropoxy)-phenyl]-4-(trifluormethyl)-2H-1-benzopyran

### a) 2-[4-(3-Chlorpropoxy)-phenyl]-7-(tetrahydropyran-2-yloxy)-3-[4-(tetrahydropyran-2-yloxy)-phenyl]-2,3-dihydro-4H-1-benzopyran-4-on

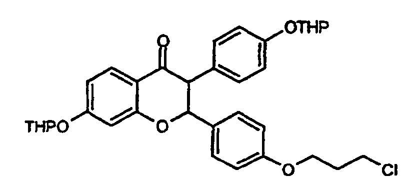

60 g 2-[4-Hydroxyphenyl]-7-(tetrahydropyran-2-yloxy)-3-[4-(tetrahydropyran-2-yloxy)-phenyl]-2,3-dihydro-4H-1-benzopyran-4-on, 91,3 g 1-Brom-3-chlorpropan und 100 g Kaliumcarbonat werden 4 Stunden unter Rückfluss in 200 ml Aceton gerührt. Nach dem Abkühlen wird der Niederschlag abgesaugt und mit Aceton gewaschen. Das Filtrat wird am Rotationsverdampfer bis zur Trockene eingeengt. Der Rückstand wird über Kieselgel mit Hexan/Essigester 9:1, 4:1, 3:1 und 7:3 chromatographiert. Es werden 49,74 g Produkt gewonnen.
MS (Cl, NH₃, 70 eV, 150°C) : m/e = 593 (M+H⁺, 35,0%), 509 (17,3%), 425 (6,4%).

### b) 2-[4-(3-Chlorpropoxy)-phenyl]- 4,7-dihydroxy-3-(4-hydroxyphenyl)-4-(trifluormethyl)-2,3-dihydro-4H-1-benzopyran

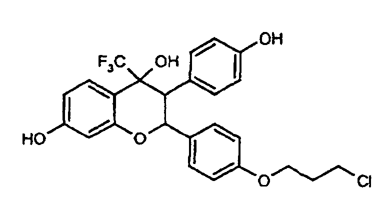

12 g 2-[4-(3-Chlorlpropoxy)-phenyl]-7-(tetrahydropyran-2-yloxy)-3-[4-(tetrahydropyran-2-yloxy)-phenyl]-2,3-dihydro-4H-1-benzopyran-4-on in 100 ml Ethylenglycoldimethylether werden mit 10,2 ml (Trifluormethyl)-trimethylsilan und einer Spatelspitze Cäsiumfluorid versetzt und bei 4 Stunden -10°C und 20 Stunden bei RT gerührt. Anschließend wird die Reaktionslösung in 100 ml gesättigte Natriumchloridlösung gegossen. Es wird dreimal mit 100 ml Essigester ausgeschüttelt. Die vereinigten organischen Phasen werden zweimal mit 50 ml Wasser gewaschen, über Magnesiumsulfat getrocknet und am Rotationsverdampfer eingeengt. Das anfallende Rohprodukt wird über Kieselgel mit Hexan und Hexan/Essigester 8:2 gereinigt. Es fallen 8,2 g Produkt an.
8,2 g 2-[4-(3-Chlorpropoxy)-phenyl]-7-hydroxy-3-(4-hydroxyphenyl)-4-(trifluormethyl)-4-(trimethylsiloxy)-2,3-dihydro-4H-1-benzopyran werden in 100 ml Methanol gelöst. Bei 10-15°C werden 40 ml 5,5 M HCl langsam zugetropft. Es wird 4 Stunden bei RT nachgerührt. Die flüchtigen Bestandteile werden am Rotationsverdampfer abgezogen, der verbleibende Rückstand wird mit 100 ml Wasser versetzt und dreimal mit 100 ml Dichlormethan extrahiert. Die vereinigten organischen Phasen werden zweimal mit 50 ml Wasser gewaschen, über Magnesiumsulfat getrocknet und am Rotationsverdampfer eingeengt. Der Rückstand wird über Kieselgel mit Hexan und Hexan/Essigester 3:1 gereinigt. Es werden 3 g Produkt isoliert.

### c) 2-[4-(3-Chlorpropoxy)-phenyl]-7-hydroxy-3-(4-hydroxyphenyl)-4-(trifluormethyl)-2H-1-benzopyran

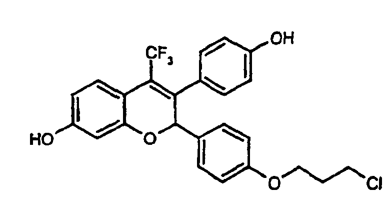

Bei 0-5°C werden zu 3 g 2-[4-(3-Chlorpropoxy)-phenyl]-4,7-dihydroxy-3-(4-hydroxyphenyl)-4-(trifluormethyl)-2,3-dihydro-4H-1-benzopyran in 80 ml THF gleichzeitig 3,34ml Trifluoressigsäureanhydrid und 3,9 ml Pyridin zugetropft. Es wird 1 Stunde bei 0-5°C und 4 Stunden bei Raumtemperatur nachgerührt, bevor 60 ml gesättigte Natriumchloridlösung zugesetzt werden. Nach dreimaliger Extraktion mit 80 ml Essigester werden die vereinigten organischen Phasen zweimal mit 50 ml Wasser gewaschen, über Magnesiumsulfat getrocknet und am Rotationsverdampfer eingeengt. Der verbleibende Rückstand ergibt nach chromatographischer Reinigung über Kieselgel mit Hexan und Hexan/Essigester 3:1 1,7 g Produkt.
MS (EI, 70 eV, 150°C) : m/e = 476 (M⁺, 9,9%), 407 (M-CF₃⁺, 36,8%), 383 (31,6%), 307 (100%).

### d) 7-Hydroxy-3-(4-hydroxyphenyl)-2-[4-(3-piperidin-1-ylpropoxy)-phenyl]-4-(trifluormethyl)-2H-1-benzopyran

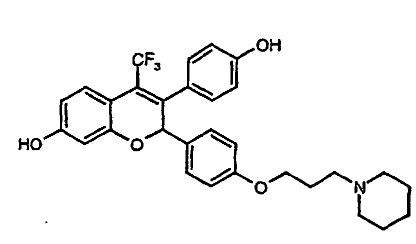

0,035 g 2-[4-(3-Chlorpropoxy)-phenyl]-7-hydroxy-3-(4-hydroxyphenyl)-4-(trifluormethyl)-2H-1-benzopyran in 1 ml 1-Methyl-2-pyrrolidon werden mit 10,1 mg Kaliumcarbonat, einer Spatelspitze Kaliumiodid und 0,036 ml Piperidin versetzt und 12 h bei 80°C gerührt. Nach Zugabe von 20 ml Essigester wird die organische Phase dreimal mit 20 ml Wasser gewaschen, über Magnesiumsulfat getrocknet und am Rotationsverdampfer eingeengt. Die Reinigung des Produktes mittels präparativer HPLC (Nucleosil 50-7, Dichlormethan/Methanol 8:2, 30 ml/min) ergibt 30 mg Produkt.

### Beispiele 18 bis 31

Analog werden die anderen Amine (5 Moläquivalente) mit 35 mg 2-[4-(2-Chlorethoxy)-phenyl]-7-hydroxy-3-(4-hydroxyphenyl)-4-(trifluormethyl)-2H-1-benzopyran umgesetzt, wobei 22 Stunden bei 80°C gerührt wird. Die Reinigung der Produkte mittels präparativer HPLC (Nucleosil 50-7, Dichlormethan/Methanol 7:3, 30 ml/min) ergibt die Produkte.

| **Beispiel** | **Amin** | **Ausbeute** |
|---|---|---|
| 18 | N,N-Dimethylamin | 20 mg |
| 19 | N,N-Diethylamin | 9 mg |
| 20 | N-Methyl-N-phenethylamin | 30 mg |
| 21 | N-Methyl-N-(3-phenylpropyl)amin | 30 mg |
| 22 | N-Butyl-N-ethylamin | 23 mg |
| 23 | Thiomorpholin | 30 mg |
| 24 | Pyrrolidin | 25 mg |
| 25 | Morpholin | 24 mg |
| 26 | N-Methyl-N-pentylamin | 22 mg |
| 27 | N-Isobutyl-N-methylamin | 16 mg |
| 28 | N-Benzyl-N-methylamin | 25 mg |
| 29 | N,N-Dipropylamin | 35 mg |
| 30 | Hexamethylenimin | 26 mg |
| 31 | N-Butyl-N-methylamin | 23 mg |

### Beispiel 32

### 7-Hydroxy-3-(4-hydroxyphenyl)-2-[4-(4-piperidin-1-ylbutoxy)-phenyl]-4-(trifluormethyl)-2H-1-benzopyran

### a) 2-[4-(4-Chlorbutoxy)-phenyl]-7-(tetrahydropyran-2-yloxy)-3-[4-(tetrahydropyran-2-yloxy)-phenyl]-2,3-dihydro-4H-1-benzopyran-4-on

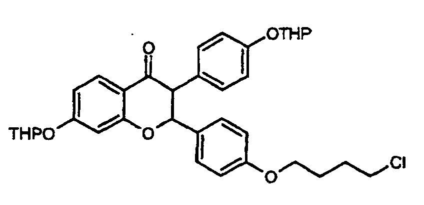

60 g 2-[4-Hydroxyphenyl]-7-(tetrahydropyran-2-yloxy)-3-[4-(tetrahydropyran-2-yloxy)-phenyl]-2,3-dihydro-4H-1-benzopyran-4-on, 99,4 g 1-Brom-4-chlorbutan und 100 g Kaliumcarbonat werden 4 Stunden unter Rückfluss in 200 ml Aceton gerührt. Nach dem Abkühlen wird der Niederschlag abgesaugt und mit Aceton gewaschen. Das Filtrat wird am Rotationsverdampfer bis zur Trockene eingeengt. Der Rückstand wird über Kieselgel mit Hexan/Essigester 9:1, 4:1, 3:1 und 7:3 gereinigt. Es fallen 22,6 g Produkt an.

### b) 2-[4-(4-Chlorbutoxy)-phenyl]-4,7-dihydroxy-3-(4-hydroxyphenyl)-4-(trifluormethyl)-2,3-dihydro-4H-1-benzopyran

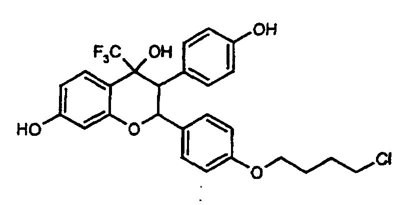

20,4 g 2-[4-(4-Chlorlbutoxy)-phenyl]-7-(tetrahydropyran-2-yloxy)-3-[4-(tetrahydropyran-2-yloxy)-phenyl]-2,3-dihydro-4H-1-benzopyran-4-on in 150 ml Ethylenglycoldimethylether werden mit 17 ml (Trifluormethyl)-trimethylsilan und einer Spatelspitze Cäsiumfluorid versetzt und 4 Stunden bei -10°C und 20 h bei RT gerührt. Anschließend wird die Reaktionslösung in 100 ml gesättigte Natriumchloridlösung gegossen. Es wird dreimal mit 100 ml Essigester ausgeschüttelt. Die vereinigten organischen Phasen werden zweimal mit 50 ml Wasser gewaschen, über Magnesiumsulfat getrocknet und am Rotationsverdampfer eingeengt. Das anfallende Rohprodukt wird über Kieselgel mit Hexan und Hexan/Essigester 8:2 gereinigt. Es werden 7,8 g Produkt isoliert.
8,8 g 2-[4-(4-Chlorbutoxy)-phenyl]-7-hydroxy-3-(4-hydroxyphenyl)-4-(trifluormethyl)-4-(trimethylsiloxy)-2,3-dihydro-4H-1-benzopyran werden in 100 ml Methanol gelöst. Bei10-15°C werden 40 ml 5,5 M HCl langsam zugetropft. Es wird 4 Stunden bei RT nachgerührt. Die flüchtigen Bestandteile werden am Rotationsverdampfer abgezogen, der verbleibende Rückstand wird mit 100 ml Wasser versetzt und dreimal mit 150 ml Dichlormethan extrahiert. Die vereinigten organischen Phasen werden zweimal mit 80 ml Wasser gewaschen, über Magnesiumsulfat getrocknet und am Rotationsverdampfer eingeengt. Nach chromatographischer Reinigung über Kieselgel mit Hexan und Hexan/Essigester 3:1 fallen 2,7 g Produkt an.

### c) 2-[4-(4-Chlorbutoxy)-phenyl]-7-hydroxy-3-(4-hydroxyphenyl)-4-(trifluormethyl)-2H-1-benzopyran

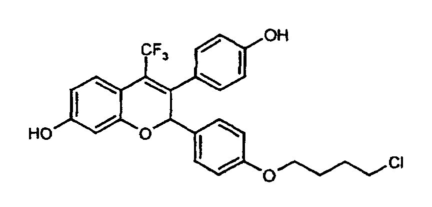

Bei 0-5°C werden gleichzeitig zu 2,7 g 2-[4-(4-Chlorbutoxy)-phenyl]-4,7-dihydroxy-3-(4-hydroxyphenyl)- 4-(trifluormethyl)-2,3-dihydro-4H-1-benzopyran in 40 ml THF 2,92ml Trifluoressigsäureanhydrid und 3,41 ml Pyridin zugetropft. Es wird 1 Stunde bei 0-5°C und 2,5 Stunden bei RT nachgerührt, bevor 40 ml gesättigte Natriumchloridlösung zugefügt werden. Es wird dreimal mit 50 ml Essigester ausgeschüttelt. Die vereinigten organischen Phasen werden über Magnesiumsulfat getrocknet und am Rotationsverdampfer eingeengt. Nach chromatographischer Reinigung des Rückstandes über Kieselgel mit Hexan und Hexan/Essigester 3:1 fallen 1,4 g Produkt an.
MS (EI, 70 eV, 150°C) : m/e = 490 (M⁺, 10,1%), 421 (M-CF₃⁺, 32,0%), 397 (22,0%), 307 (100%).

### d) 7-Hydroxy-3-(4-hydroxyphenyl)-2-[4-(4-piperidin-1-ylbutoxy)-phenyl]-4-(trifluormethyl)-2H-1-benzopyran

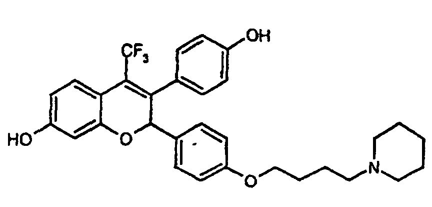

0,036 g 2-[4-(3-Chlorpropoxy)-phenyo-7-hydroxy-3-(4-hydroxyphenyl)-4-(trifluormethyl)-2H-1-benzopyran in 1 ml 1-Methyl-2-pyrrolidon werden mit 10,1 mg Kaliumcarbonat, einer Spatelspitze Kaliumiodid und 0,036 ml Piperidin versetzt und 12 h bei 80°C gerührt. Nach Zugabe von 20 ml Essigester wird die organische Phase dreimal mit 20 ml Wasser gewaschen, über Magnesiumsulfat getrocknet und am Rotationsverdampfer eingeengt. Die Reinigung des Produktes mittels präparativer HPLC (Nucleosil 50-7, Dichlormethan/Methanol 8:2, 30 ml/min) ergibt 26 mg Produkt.

### Beispiele 33 bis 46

Analog werden die anderen Amine (5 Moläquivalente) mit 36 mg 2-[4-(2-Chlorethoxy)-phenyl]-7-hydroxy-3-(4-hydroxyphenyl)-4-(trifluormethyl)-2H-1-benzopyran umgesetzt, wobei 22 Stunden bei 80°C gerührt wird. Die Reinigung der Produkte mittels präparativer HPLC (Nucleosil 50-7, Dichlormethan/Methanol 7:3, 30 ml/min) ergibt die Produkte.

| **Beispiel** | **Amin** | **Ausbeute** |
|---|---|---|
| 33 | N,N-Dimethylamin | 20 mg |
| 34 | N,N-Diethylamin | 9 mg |
| 35 | N-Methyl-N-phenethylamin | 23 mg |
| 36 | N-Methyl-N-(3-phenylpropyl)amin | 23 mg |
| 37 | N-Butyl-N-ethylamin | 18 mg |
| 38 | Thiomorpholin | 28 mg |
| 39 | Pyrrolidin | 27 mg |
| 40 | Morpholin | 18 mg |
| 41 | N-Methyl-N-pentylamin | 27 mg |
| 42 | N-Isobutyl-N-methylamin | 17 mg |
| 43 | N-Benzyl-N-methylamin | 24 mg |
| 44 | N,N-Dipropylamin | 13 mg |
| 45 | Hexamethylenimin | 25 mg |
| 46 | N-Butyl-N-methylamin | 22 mg |

### Beispiel 47

### (+)-7-Methoxy-3-(4-methoxyphenyl)-2-[4-(2-piperidin-1-ylethoxy)-phenyl]-4-(trifluormethyl)-2H-1-benzopyran

### Beispiel 48

### (-)-7-Methoxy-3-(4-methoxyphenyl)-2-[4-(2-piperidin-1-ylethoxy)-phenyl]-4-(trifluormethyl)-2H-1-benzopyran

### a) 2-[4-(2-Chlorethoxy)-phenyl]-7-methoxy-3-(4-methoxyphenyl)-4-(trifluormethyl)-2H-1-benzopyran

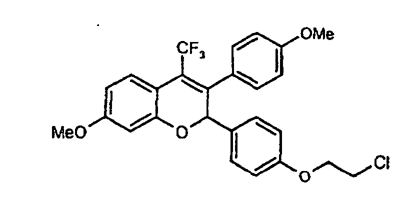

170 mg 2-[4-(2-Chlorethoxy)-phenyl]-7-hydroxy-3-(4-hydroxyphenyl)-4-(trifluormethyl)-2H-1-benzopyran in 5 ml Aceton werden mit 0,0297 ml Methyliodid und 101 mg Kaliumcarbonat versetzt und über Nacht bei RT gerührt. Die Reaktionsmischung wird in verdünnte Natriumchloridlösung gegossen und dreimal mit Essigester extrahiert. Die vereinigten organischen Phasen werden einmal mit Wasser und einmal mit gesättigter Natriumchloridlösung gewaschen, über Magnesiumsulfat getrocknet und im Vakuum eingeengt. Die Reinigung erfolgt über präparative Dünnschichtplatten mit Hexan/Essigester 1:1.Es werden 52 mg Produkt isoliert.

### b) 7-Methoxy-3-(4-methoxyphenyl)-2-[4-(2-piperidin-1-ylethoxy)-phenyl]-4-(trifluormethyl)-2H-1-benzopyran

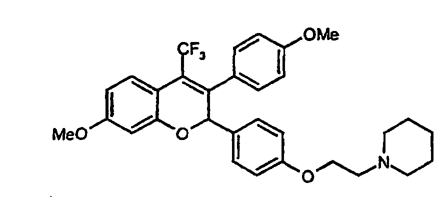

125 mg 2-[4-(2-Chlorethoxy)-phenyl]-7-methoxy-3-(4-methoxyphenyl)-4-(trifluormethyl)-2H-1-benzopyran und 12,5 mg Kaliumiodid werden in 2,5 ml Piperidin 18 Stunden bei 100°C gerührt. Die abgekühlte Lösung wird in verdünnte Natriumchloridlösung gegossen, die wässrige Phase wird dreimal mit Essigester extrahiert, und die vereinigten organischen Phasen werden über Magnesiumsulfat getrocknet und im Vakuum eingeengt. Das Produkt wird über analytische Dünnschichtplatten mit Dichlormethan/Methanol 9:1 gereinigt. Es fallen 86 mg Produkt an, die mit Hilfe chiraler HPLC (Chiralpak AD 10µ, 250 x 60 mm, Hexan (0,1% Triethylamin) : Ethanol (0,1% Triethylamin) 9:1, 90 ml/min) in die Enantiomeren aufgetrennt werden.
Vom (+)-Enantiomer werden 22 mg und vom (-)-Enantiomer 30 mg gewonnen.
(+)-Enantiomer: [α]D = +286,0° (0,48 in CHCl₃)
(-)-Enantiomer: [α]D = -241,9° (0,54 in CHCl₃)

### Beispiel 49

### 7-Hydroxy-3-(4-hydroxyphenyl)-2-[4-(3-piperidin-1-yl-1-thiapropyl)-phenyl]-4-(trifluormethyl)-2H-1-benzopyran

### a) 4-(Methylsulfinyl)-benzaldehyd

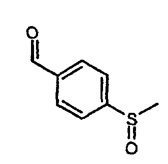

Bei 0°C werden zu 10,52 g 4-Methylthio-benzaldehyd in 197 ml Chloroform 16,19 g 3-Chlorperbenzoesäure (70%ig) zugegeben. Nach 1 Stunde bei 0°C werden 7,30 g Calciumhydroxid zugesetzt. Es wird auf RT erwärmt und 30 Minuten bei RT nachgerührt. Die Suspension wird abgesaugt und zweimal mit Dichlomethan gewaschen. Die vereinigten organischen Phasen werden zur Trockene eingeengt und über Kieselgel mit Dichlormethan, Dichlormethan/Methanol 99:1, 98:2 und 97:3 gereinigt. Es fallen 9,14 g weißer Feststoff an.

### b) 4-Mercaptobenzaldehyd

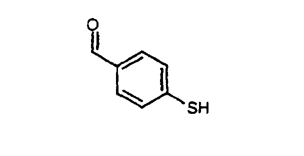

4,8 g 4-(Methylsulfinyl)-benzaldehyd werden in 50 ml Trifluoracetanhydrid gelöst und 30 Minuten unter Rückfluss gerührt. Die leichtflüchtigen Bestandteile werden abgezogen, und der Rückstand wird in 200 ml Triethylamin gegeben, wozu anschließend noch 200 ml Methanol zugesetzt werden. Es wird kurz durchgerührt und anschließend werden die leichtflüchtigen Bestandteile abgezogen. Der Rückstand wird mit Dichlormethan verdünnt, zweimal mit gesättigter Ammoniumchloridlösung gewaschen, über Magnesiumsulfat getrocknet und am Rotationsverdampfer eingeengt. Es fallen 4,52 g gelber Feststoff an.

### c) 4-(3-Chlor-1-thiapropyl)-benzaldehyd

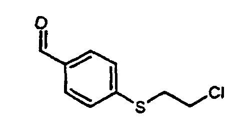

4,51 g 4-Mercaptobenzaldehyd in 43,8 ml Acetonitril werden mit 7,00 g Kaliumcarbonat und 11,8 ml 1-Brom-2-chlorethan versetzt und 4 Stunden bei 90°C gerührt. Es wird mit Essigester verdünnt, zweimal mit Wasser und einmal mit gesättigter Natriumchloridlösung gewaschen. Die vereinigten organischen Phasen werden über Magnesiumsulfat getrocknet und eingeengt. Das Rohprodukt wird über Kieselgel mit Hexan, Hexan/Essigester 95:5, 90:10 und 85:15 gereinigt. Es werden 3,88 g gelbe Flüssigkeit gewonnen.

### d) 2-[4-(3-Chlor-1-thiapropyl)-phenyl]-7-(tetrahydropyran-2-yloxy)-3-[4-(tetrahydropyran-2-yloxy)-phenyl]-2,3-dihydro-4H-1-benzopyran-4-on

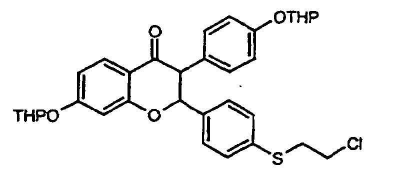

Zu 8,91 g 1-[2-Hydroxy-4-(tetrahydropyran-2-yloxy)-phenyl]-2-[4-(tetrahydropyran-2-yloxy)-phenyl]-ethanon und 4,77 g 4-(3-Chlor-1-thiapropyl)-benzaldehyd in 78,6 ml Toluol werden 0,65 ml Piperidin gegeben. Es wird 5,5 Stunden unter Rückfluss am Wasserabscheider gerührt. Die Lösung wird abgekühlt und bis zur Trockene eingeengt. Das Rohprodukt wird über Kieselgel mit Hexan und Hexan/Essigester 95:5, 90:10, 85:15, 80:20, 75:25 und 70:30 gereinigt. Vom Produkt fallen 1.512 g als gelber Schaum an. Daneben werden noch 3,70 g des entsprechenden Chalkons isoliert, das nach nachfolgender Prozedur in das Produkt überführt wird.
Bei RT werden 4,69 g des Chalkons in 205 ml Methanol gelöst und mit 32,05 g Natriumacetat versetzt. Es wird 6 Stunden bei 100°C Badtemperatur gerührt. Nach dem Erkalten wird das Solvens am Rotationsverdampfer abgezogen, der Rückstand wird in Wasser gegossen und dreimal mit Essigester extrahiert. Die vereinigten organischen Phasen werden zweimal mit Wasser gewaschen, über Magnesiumsulfat getrocknet und am Rotationsverdampfer eingeengt. Säulenchromatographische Reinigung über Kieselgel mit Hexan und Hexan/Essigester 9:1, 8:2, 7:3 und 6:4 ergibt 1,408 g eines hellen Schaumes.
MS (Cl, NH₃, 70 eV, 150°C) : m/e = 595 (M+H⁺, 18,7%), 511 (20,9%).

### e) 2-[4-(3-Chlor-1-thiapropyl)-phenyl]-4,7-dihydroxy-3-(4-hydroxyphenyl)-4-(trifluormethyl)-2,3-dihydro-4H-1-benzopyran

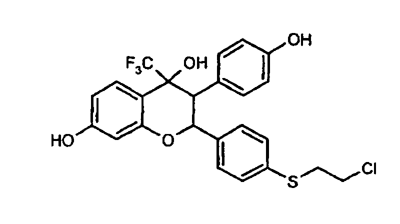

Bei -10°C werden zu 2,04 g 2-[4-(3-Chlor-1-thiapropyl)-phenyl]-7-(tetrahydropyran-2-yloxy)-3-[4-(tetrahydropyran-2-yloxy)-phenyl]-2,3-dihydro-4H-1-benzopyran-4-on in 19,6 ml Ethylenglycoldimethylether 2,54 ml (Trifluormethyl)-trimethylsilan und 31,2 mg Cäsiumfluorid zugegeben und bei -10°C 1 Stunde gerührt. Anschließend wird die Reaktionslösung in ml gesättigte Natriumchloridlösung gegossen. Es wird dreimal mit Ether ausgeschüttelt. Die vereinigten organischen Phasen werden über Magnesiumsulfat getrocknet und am Rotationsverdampfer eingeengt. Das anfallende Rohprodukt wird über Kieselgel mit Hexan und Hexan/Essigester 95:5, 90:10 und 85:15 gereinigt. Es werden 0,825 g gewonnen.
0,811 g 2-[4-(3-Chlor-1-thiapropyl)-phenyl]-7-hydroxy-3-(4-hydroxyphenyl)-4-(trifluormethyl)-4-(trimethylsiloxy)-2,3-dihydro-4H-1-benzopyran werden in 21,8 ml Methanol gelöst und mit 6,2 ml 5,5 M HCl 0,5 Stunden bei RT gerührt. Die flüchtigen Bestandteile werden am Rotationsverdampfer abgezogen, der verbleibende Rückstand wird in Wasser gegeben und dreimal mit Dichlormethan extrahiert. Die vereinigten organischen Phasen werden zweimal mit Wasser gewaschen, über Magnesiumsulfat getrocknet und am Rotationsverdampfer eingeengt. Der Rückstand wird über Kieselgel mit Hexan und Hexan/Essigester 9:1, 8:2, 7:3, 6:4 und 1:1 gereinigt. Es werden 448 mg Produkt gewonnen.
MS (EI, 70 eV, 150°C) : rn/e = 496 (M⁺, 4,2%), 427 (M-CF₃⁺, 1,1%), 409 (1,2%), 290 (100%).

### f) 2-[4-(3-Chlor-1-thiapropyl)-phenyl]-7-hydroxy-3-(4-hydroxyphenyl)-4-(trifluormethyl)-2H-1-benzopyran

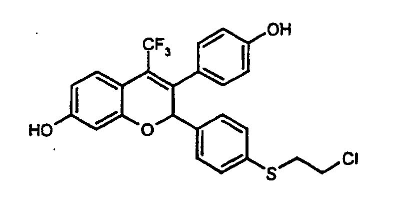

Auf dem Eisbad werden zu 448 mg 2-[4-(3-Chlor-1-thiapropyl)-phenyl]-4,7-dihydroxy-3-(4-hydroxyphenyl)- 4-(trifluormethyl)-2,3-dihydro-4H-1-benzopyran in 9,09 ml THF gleichzeitig 0,501 ml Trifluoressigsäureanhydrid und 0,723 ml Pyridin zugetropft. Es wird 2,5 Stunden bei Raumtemperatur nachgerührt, bevor auf gesättigte Natriumchloridlösung gegossen wird. Es wird dreimal mit Essigester ausgeschüttelt Die vereinigten organischen Phasen werden über Magnesiumsulfat getrocknet und am Rotationsverdampfer eingeengt Der verbleibende Rückstand wird über Kieselgel mit Hexan/Essigester 7/3 gereinigt. Es fallen 415 mg Produkt an.
MS (EI, 70 eV, 150°C) : m/e = 478 (M⁺, 12,9%), 409 (M-CF₃⁺, 24,6%), 385 (16,4%), 307 (100%).

### g) 7-Hydroxy-3-(4-hydroxyphenyl)-2-[4-(3-piperidin-1-yl-1-thiapropyl)-phenyl]-4-(trifluormethyl)-2H-1-benzopyran

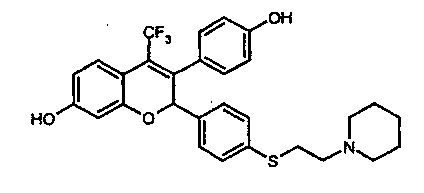

220 mg 2-[4-(3-Chlor-1-thiapropyl)-phenyl]-7-hydroxy-3-(4-hydroxyphenyl)-4-(trifluormethyl)-2H-1-benzopyran in 2 ml Piperidin werden mit 42,7 mg Kaliumiodid versetzt und 6 h bei 100°C gerührt. Nach dem Erkalten wird auf Wasser gegeossen und dreimal mit Essigester ausgeschüttelt. Die vereinigten organischen Phasen werden zweimal mit Wasser gewaschen, über Magnesiumsulfat getrocknet und am Rotationsverdampfer eingeengt. Die Reinigung des Produktes erfolgt über Kieselgel mit Dichlormethan/Methanol 9:1. Es fallen 274 mg Produkt an.
MS (EI, 70 eV, 150°C) : m/e = 527 (M⁺, 0,8%), 307 (1,9%).

Weitere erfindungsgemäße Thiaverbindungen lassen sich durch analoge Vorgehensweise herstellen, indem in der Stufe c) ein längeres α,ω-Dihalogenalkan eingesetzt wird.

## Patentansprüche

1. 4-Trifluormethyl-2H-benzopyrane der allgemeinen Formel I 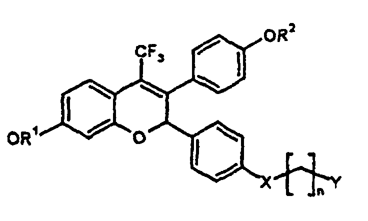 worin
R¹ und R² unabhängig voneinander
ein Wasserstoffatom,
eine Methylgruppe,
eine gerad- oder verzweigtkettige C₂-C₅-Alkylgruppe,
eine gerad- oder verzweigtkettige Alkanoylgruppe mit bis zu 5 Kohlenstoffatomen oder eine Benzoylgruppe,
X ein Sauerstoff- oder Schwefelatom oder eine Methylengruppe
n 2, 3 oder 4,
Y ein Piperidin-, Pyrrolidin-, Hexamethylenimino-, Dimethylamino-, Diethylamino-, Dipropylamino-, N-Methyl-N-phenethylamino-, N-Methyl-N-(3-phenylpropyl)amino-, N-Butyl-N-ethylamino-, Thiomorpholino-, Morpholino-, N-Methyl-N-pentylamino-, N-Isobutyl-N-methylamino-, N-Benzyl-N-methylamino-, N-Butyl-N-methylaminorest
oder
eine Gruppe -NR³R⁴, worin R³ und R⁴ unabhängig voneinander
ein Wasserstoffatom; eine Monofluormethyl-, Difluormethyl-, Trifluormethyl-, Pentafluorethyl-, 2,2,2-Trifluorethyl-, 4,4,4-Trifluorbutyl-, 3,3,4,4,4-Pentafluorbutyl-, 4,4,5,5,5-Pentafluorpentyl-, Nonafluorbutylgruppe
bedeuten,
sowie deren physiologisch verträgliche Additionssalze mit organischen und anorganischen Säuren.

2. Verbindungen der allgemeinen Formel I nach Anspruch 1 in Form ihrer Racemate.

3. Verbindungen der allgemeinen Formel I nach Anspruch 1 in Form der getrennten optischen Isomeren.

4. Verbindungen der allgemeinen Formel I nach einem der vorherigen Ansprüche, worin R¹ und/oder R² ein Pivaloylrest ist.

5. (+)-7-Hydroxy-3-(4'-hydroxyphenyl)-2-[4"-(2"'-piperidin-1-ylethoxy)-phenyl]-4-(trifluormethyl)-2H-1-benzopyran,
(-)-7-Hydroxy-3-(4'-hydroxyphenyl)-2-[4"-(2"'-piperidin-1-ylethoxy)-phenyl]-4-(trifluormethyl)-2H-1-benzopyran,
(+)-7-Pivaloyloxy-3-(4'-pivaloyloxyphenyl)-4-trifluormethyl-2-(4"-(2"'-piperidinoethoxy)phenyl)-2H-benzopyran,
(-)-7-Pivaloyloxy-3-(4'-pivaloyloxyphenyl)-4-trifluormethyl-2-(4"-(2"'-piperidinoethoxy)-phenyl)-2H-benzopyran,
2-[4"-(2"'-Dimethylaminoethoxy)-phenyl]-7-hydroxy-3-(4'-hydroxyphenyl)-4-(trifluormethyl)-2H-1-benzopyran,
2-[4"-(2"'-Diethylaminoethoxy)-phenyl]-7-hydroxy-3-(4'-hydroxyphenyl)-4-(trifluormethyl)-2H-1-benzopyran,
7-Hydroxy-3-(4'-hydroxyphenyl)-2-[4"-(2"'-N-methyl-N-phenethylaminoethoxy)-phenyl]-4-(trifluormethyl)-2H-1-benzopyran,
7-Hydroxy-3-(4'-hydroxyphenyl)-2-{4"-[2"'-N-methyl-N-(3""phenylpropyl)aminoethoxy]-phenyl}-4-(trifluormethyl)-2H-1-benzopyran,
2-[4"-(2"'-N-Butyl-N-ethylaminoethoxy)-phenyl]-7-hydroxy-3-(4'-hydroxyphenyl)-4-(trifluormethyl)-2H-1-benzopyran,
7-Hydroxy-3-(4'-hydroxyphenyl)-2-[4"-(thiomorpholin-1-ylethoxy)-phenyl]-4-(trifluormethyl)-2H-1-benzopyran,
7-Hydroxy-3-(4'-hydroxyphenyl)-2-[4"-(2"'-pyrrolidin-1-ylethoxy)-phenyl]-4-(trifluormethyl)-2H-1-benzopyran,
7-Hydroxy-3-(4'-hydroxyphenyl)-2-[4"-(2"'-morpholin-1-ylethoxy)-phenyl]-4-(trifluormethyl)-2H-1-benzopyran,
7-Hydroxy-3-(4'-hydroxyphenyl)-2-[4"-(2"'-N-methyl-N-pentylaminoethoxy)-phenyl]-4-(trifluormethyl)-2H-1-benzopyran,
7-Hydroxy-3-(4'-hydroxyphenyl)-2-[4"-(2"'-N-isobutyl-N-methylaminoethoxy)-phenyl]-4-(trifluormethyl)-2H-1-benzopyran,
2-[4"-(2"'-N-Benzyl-N-methylaminoethoxy)-phenyl]-7-hydroxy-3-(4'-hydroxyphenyl)-4-(trifluormethyl)-2H-1-benzopyran,
2-[4"-(2"'-N,N-Di-n-propylaminoethoxy)-phenyl]-7-hydroxy-3-(4'-hydroxyphenyl)-4-(trifluormethyl)-2H-1-benzopyran,
2-[4"-(2"'-Hexamethylenimin-1-ylethoxy)-phenyl]-7-hydroxy-3-(4'-hydroxyphenyl)-4-(trifluormethyl)-2H-1-benzopyran,
2-[4"-(2"'-N-Butyl-N-methylaminoethoxy)-phenyl]-7-hydroxy-3-(4'-hydroxyphenyl)-4-(trifluormethyl)-2H-1-benzopyran,
7-Hydroxy-3-(4'-hydroxyphenyl)-2-[4"-(3"'-piperidin-1-ylpropoxy)-phenyl]-4-(trifluormethyl)-2H-1-benzopyran,
2-[4"-(3"'-N-Dimethylaminopropoxy)-phenyl]-7-hydroxy-3-(4'-hydroxyphenyl)-4-(trifluormethyl)-2H-1-benzopyran,
2-[4"-(3"'-N-Diethylaminopropoxy)-phenyl]-7-hydroxy-3-(4'-hydroxyphenyl)-4-(trifluormethyl)-2H-1-benzopyran,
7-Hydroxy-3-(4'-hydroxyphenyl)-2-[4"-(3"'-N-methyl-N-phenethylaminopropoxy)-phenyl]-4-(trifluormethyl)-2H-1-benzopyran,
7-Hydroxy-3-(4'-hydroxyphenyl)-2-{4"-[3"'-N-methyl-N-(3""phenylpropyl)aminopropoxy)-phenyl}-4-(trifluormethyl)-2H-1-benzopyran,
2-[4"-(3"'-N-Butyl-N-ethylaminopropoxy)-phenyl]-7-hydroxy-3-(4'-hydroxyphenyl)-4-(trifluormethyl)-2H-1-benzopyran,
7-Hydroxy-3-(4'-hydroxyphenyl)-2-[4"-(3"'-thiomorpholin-1-ylpropoxy)-phenyl]-4-(trifluormethyl)-2H-1-benzopyran,
7-Hydroxy-3-(4'-hydroxyphenyl)-2-[4"-(3"'-pyrrolidin-1-ylpropoxy)-phenyl]-4-(trifluormethyl)-2H-1-benzopyran,
7-Hydroxy-3-(4'-hydroxyphenyl)-2-[4"-(3"'-morpholin-1-ylpropoxy)-phenyl]-4-(trifluormethyl)-2H-1-benzopyran,
7-Hydroxy-3-(4'-hydroxyphenyl)-2-[4"-(3"'-N-methyl-N-pentylaminopropoxy)-phenyl]-4-(trifluormethyl)-2H-1-benzopyran,
7-Hydroxy-3-(4'-hydroxyphenyl)-2-[4"-(3"'-N-isobutyl-N-methylaminopropoxy)-phenyl]-4-(trifluormethyl)-2H-1-benzopyran,
2-[4"-(3"'-N-Benzyl-N-methylaminopropoxy)-phenyl]-7-hydroxy-3-(4'-hydroxyphenyl)-4-(trifluormethyl)-2H-1-benzopyran,
2-[4"-(3"'-N,N-Di-n-propylaminopropoxy)-phenyl]-7-hydroxy-3-(4'-hydroxyphenyl)-4-(trifluormethyl)-2H-1-benzopyran,
2-[4"-(3"'-Hexamethylenimin-1-ylpropoxy)-phenyl]-7-hydroxy-3-(4'-hydroxyphenyl)-4-(trifluormethyl)-2H-1-benzopyran,
2-[4"-(3"'-N-Butyl-N-methylaminopropoxy)-phenyl]-7-hydroxy-3-(4'-hydroxyphenyl)-4-(trifluormethyl)-2H-1-benzopyran,
7-Hydroxy-3-(4'-hydroxyphenyl)-2-[4"-(4"'-piperidin-1-ylbutoxy)-phenyl]-4-(trifluormethyl)-2H-1-benzopyran,
2-[4"-(4"'-N-Dimethylaminobutoxy)-phenyl]-7-hydroxy-3-(4'-hydroxyphenyl)-4-(trifluormethyl)-2H-1-benzopyran,
2-[4"-(4"'-N-Diethylaminobutoxy)-phenyl]-7-hydroxy-3-(4'-hydroxyphenyl)-4-(trifluormethyl)-2H-1-benzopyran,
7-Hydroxy-3-(4'-hydroxyphenyl)-2-[4"-(4"'-N-methyl-N-phenethylaminobutoxy)-phenyl]-4-(trifluormethyl)-2H-1-benzopyran,
7-Hydroxy-3-(4'-hydroxyphenyl)-2-{4"-[4"'-N-methyl-N-(3""phenylpropyl)aminobutoxy]-phenyl}-4-(trifluormethyl)-2H-1-benzopyran,
2-[4"-(4"'-N-Butyl-N-ethylaminobutoxy)-phenyl]-7-hydroxy-3-(4'-hydroxyphenyl)-4-(trifluormethyl)-2H-1-benzopyran,
7-Hydroxy-3-(4'-hydroxyphenyl)-2-[4"-(4"'-thiomorpholin-1-ylbutoxy)-phenyl]-4-(trifluormethyl)-2H-1-benzopyran,
7-Hydroxy-3-(4'-hydroxyphenyl)-2-[4"-(4"'-pyrrolidin-1-ylbutoxy)-phenyl]-4-(trifluormethyl)-2H-1-benzopyran,
7-Hydroxy-3-(4'-hydroxyphenyl)-2-[4"-(4"'-morpholin-1-ylbutoxy)-phenyl]-4-(trifluormethyl)-2H-1-benzopyran,
7-Hydroxy-3-(4'-hydroxyphenyl)-2-[4"-(4"'-N-methyl-N-pentylaminobutoxy)-phenyl]-4-(trifluormethyl)-2H-1-benzopyran,
7-Hydroxy-3-(4'-hydroxyphenyl)-2-[4"-(4"'-N-isobutyl-N-methylaminobutoxy)-phenyl]-4-(trifluormethyl)-2H-1-benzopyran,
2-[4"-(4"'-N-Benzyl-N-methylaminobutoxy)-phenyl]-7-hydroxy-3-(4'-hydroxyphenyl)-4-(trifluormethyl)-2H-1-benzopyran,
2-[4"-(4"'-N,N-Di-n-propylaminobutoxy)-phenyl]-7-hydroxy-3-(4'-hydroxyphenyl)-4-(trifluormethyl)-2H-1-benzopyran,
2-[4"-(4"'-Hexamethylenimin-1-ylbutoxy)-phenyl]-7-hydroxy-3-(4'-hydroxyphenyl)-4-(trifluormethyl)-2H-1-benzopyran,
2-[4"-(4"'-N-Butyl-N-methylaminobutoxy)-phenyl]-7-hydroxy-3-(4'-hydroxyphenyl)-4-(trifluormethyl)-2H-1-benzopyran,
(+)-7-Methoxy-3-(4'-methoxyphenyl)-2-[4"-(2"'-piperidin-1-ylethoxy)-phenyl]-4-(trifluormethyl)-2H-1-benzopyran,
(-)-7-Methoxy-3-(4'-methoxyphenyl)-2-[4"-(2"'-piperidin-1-ylethoxy)-phenyl]-4-(trifluormethyl)-2H-1-benzopyran,
7-Hydroxy-3-(4'-hydroxyphenyl)-2-[4"-(3"'-piperidin-1-yl-1-thiapropyl)-phenyl]-4-(trifluormethyl)-2H-1-benzopyran.

6. Verbindungen nach Anspruch 5 in Form ihrer Racemate.

7. Verbindungen nach Anspruch 5 in Form der getrennten optischen Isomeren.

8. Verbindungen nach einem der vorherigen Ansprüche in Form des Hydrochlorids.

9. Pharmazeutische Präparate, enthaltend mindestens eine Verbindung der allgemeinen Formel I nach einem der vorherigen Ansprüche sowie einen pharmazeutisch verträglichen Träger.

10. Verwendung der Verbindungen der allgemeinen Formel I nach einem der vorherigen Ansprüche zur Herstellung von Arzneimitteln.

## Claims

1. 4-Trifluoromethyl-2H-benzopyrans of the general formula I 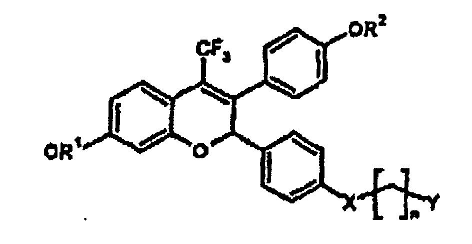 in which
R¹ and R² are independently of one another a hydrogen atom,
a methyl group,
a straight- or branched-chain C₂-C₅ alkyl group, a straight- or branched-chain alkanoyl group having up to 5 carbon atoms or a benzoyl group,
X is an oxygen or sulphur atom or a methylene group,
n is 2, 3 or 4,
Y is a piperidine, pyrrolidine, hexamethylene-imino, dimethylamino, diethylamino, dipropylamino, N-methyl-N-phenethylamino, N-methyl-N-(3-phenylpropyl)amino, N-butyl-N-ethylamino, thiom orpholino, morpholino, N-methyl-N-pentylamino, N -isobutyl-N-methyl-amino, N-benzyl-N-methyl amino, N -butyl-N-methylamino radical
or
a g roup -NR³R⁴, in which R³ and R⁴ are independently of one another a hydrogen atom;
a monofluoromethyl, difluoromethyl, trifluoromethyl, pentafluoroethyl, 2,2,2-trifluoroethyl, 4,4,4-trifluorobutyl, 3,3,4,4,4-pentafluorobutyl, 4,4,5,5,5-pentafluoropentyl, nonafluorobutyl group,
and the physiologically tolerated addition salts thereof with organic and inorganic acids.

2. Compounds of the general formula I according to Claim 1 in the form of their racemates.

3. Compounds of the general formula I according to Claim 1 in the form of the separated optical isomers.

4. Compounds of the general formula I according to any of the preceding claims, in which R ¹ and/or R² is a pivaloyl radical.

5. (+)-7-hydroxy-3-(4'-hydroxyphenyl)-2-[4"-(2"'-piperidin-1-ylethoxy)phenyl]-4-(trifluoromethyl)-2H-1-benzopyran,
(-)-7-hydroxy-3-(4'-hydroxyphenyl)-2-[4"-(2"'-piperidin-1-ylethoxy)phenyl]-4-(trifluoromethyl)-2H-1-benzopyran,
(+)-7-pivaloyloxy-3-(4'-pivaloyloxyphenyl)-4-trifluoromethyl-2-(4"-(2"'-piperidinoethoxy)phenyl)-2H-benzopyran,
(-)-7-pivaloyloxy-3-(4'-pivaloyloxyphenyl)-4-trifluoromethyl-2-(4"-(2"'-piperidinoethoxy)phenyl)-2H-benzopyran,
2-[4"-(2"'-dimethylaminoethoxy)phenyl]-7-hydroxy-3-(4'-hydroxyphenyl)-4-(trifluoromethyl)-2H-1-benzopyran
2-[4"-(2"'-diethylaminoethoxy)phenyl]-7-hydroxy-3-(4'-hydroxyphenyl)-4-(trifluoromethyl)-2H-1-benzopyran
7-hydroxy-3-(4'-hydroxyphenyl)-2-[4"-(2"'-N-methyl-N-phenethylaminoethoxy)phenyl]-4-(trifluoromethyl)-2H-1-benzopyran,
7-hydroxy-3-(4'-hydroxyphenyl)-2-{4"-(2"'-N-methyl-N-(3""-phenylpropyl)aminoethoxy]phenyl}-4-(trifluoromethyl)-2H-1-benzopyran,
2-[4"-(2"'-N-butyl-N-ethylaminoethoxy)phenyl]-7-hydroxy-3-(4'-hydroxyphenyl)-4-(trifluoromethyl)-2H-1-benzopyran,
7-hydroxy-3-(4'-hydroxyphenyl)-2-[4"-(thiomorpholin-1-ylethoxy)phenyl]-4-(trifluoromethyl)-2H-1-benzopyran
7-hydroxy-3-(4'-hydroxyphenyl)-2-[4"-(2"'-pyrrolidin-1-ylethoxy)phenyl]-4-(trifluoromethyl)-2H-1-benzopyran
7-hydroxy-3-(4'-hydroxyphenyl)-2-[4"-(2"'-morpholin-1-ylethoxy)phenyl]-4-(trifluoromethyl)-2H-1-benzopyran
7-hydroxy-3-(4'-hydroxyphenyl)-2-[4"-(2"'-N-methyl-N-pentylaminoethoxy)phenyl]-4-(trifluoromethyl)-2H-1-benzopyran,
7-hydroxy-3-(4'-hydroxyphenyl)-2-[4"-(2"'-N-isobutyl-N-methylaminoethoxy)phenyl]-4-(trifluoromethyl)-2H-1-benzopyran,
2-[4"-(2"'-N-benzyl-N-methylaminoethoxy)phenyl]-7-hydroxy-3-(4'-hydroxyphenyl)-4-(trifluoromethyl)-2H-1-benzopyran,
2-[4"-(2"'-N,N-di-n-propylaminoethoxy)phenyl]-7-hydroxy-3-(4'-hydroxyphenyl)-4-(trifluoromethyl)-2H-1-benzopyran,
2-[4"-(2"'-hexamethylenimin-1-ylethoxy)phenyl]-7-hydroxy-3-(4'-hydroxyphenyl)-4-(trifluoromethyl)-2H-1-benzopyran,
2-[4"-(2"'-N-butyl-N-methylaminoethoxy)phenyl]-7-hydroxy-3-(4'-hydroxyphenyl)-4-(trifluoromethyl)-2H-1-benzopyran,
7-hydroxy-3-(4'-hydroxyphenyl)-2-[4"-(3"'-piperidin-1-ylpropoxy)phenyl]-4-(trifluoromethyl)-2H-1-benzopyran
2-[4"-(3"'-N-dimethylaminopropoxy)phenyl]-7-hydroxy-3-(4'-hydroxyphenyl)-4-(trifluoromethyl)-2H-1-benzopyran
2-[4"-(3"'-N-diethylaminopropoxy)phenyl]-7-hydroxy-3-(4'-hydroxyphenyl)-4-(trifluoromethyl)-2H-1-benzopyran
7-hydroxy-3-(4'-hydroxyphenyl)-2-[4"-(3"'-N-methyl-N-phenethylaminopropoxy)phenyl]-4-(trifluoromethyl)-2H-1-benzopyran,
7-hydroxy-3-(4'-hydroxyphenyl)-2-{4"-[3"'-N-methyl-N-(3""-phenylpropyl)aminopropoxy]phenyl}-4-(trifluoromethyl)-2H-1-benzopyran,
2-[4"-(3"'-N-Butyl-N-ethylaminopropoxy)phenyl]-7-hydroxy-3-(4'-hydroxyphenyl)-4-(trifluoromethyl)-2H-1-benzopyran
7-hydroxy-3-(4'-hydroxyphenyl)-2-[4"-(3"'-thiomorpholin-1-ylpropoxy)phenyl-4-(trifluoromethyl)-2H-1-benzopyran
7-hydroxy-3-(4'-hydroxyphenyl)-2-[4"-(3"'-pyrrolidin-1-ylpropoxy)phenyl]-4-(trifluoromethyl)-2H-1-benzopyran
7-hydroxy-3-(4'-hydroxyphenyl)-2-[4"-(3"'-morpholin-1-ylpropoxy)phenyl-4-(trifluoromethyl)-2H-1-benzopyran
7-hydroxy-3-(4'-hydroxyphenyl)-2-[4"-(3"'-N-methyl-N-pentylaminopropoxy)phenyl]-4-(trifluoromethyl)-2H-1-benzopyran
7-hydroxy-3-(4'-hydroxyphenyl)-2-[4"-(3"'-N-isobutyl-N-methylaminopropoxy)phenyl]-4-(trifluoromethyl)-2H-1-benzopyran
2-[4"-(3"'-N-Benzyl-N-methylaminopropoxy)phenyl]-7-hydroxy-3-(4'-hydroxyphenyl)-4-(trifluoromethyl)-2H-1-benzopyran
2-[4"-(3"'-N,N-Di-n-propylaminopropoxy)phenyl]-7-hydroxy-3-(4'-hydroxyphenyl)-4-(trifluoromethyl)-2H-1-benzopyran
2-[4"-(3"'-Hexamethylenimin-1-ylpropoxy)phenyl]-7-hydroxy-3-(4'-hydroxyphenyl)-4-(trifluoromethyl)-2H-1-benzopyran
2-[4"-(3"'-N-Butyl-N-methylaminopropoxy)phenyl]-7-hydroxy-3-(4'-hydroxyphenyl)-4-(trifluoromethyl)-2H-1-benzopyran
7-hydroxy-3-(4'-hydroxyphenyl)-2-[4"-(4"'-piperidin-1-ylbutoxy)phenyl]-4-(trifluoromethyl)-2H-1-benzopyran
2-[4"-(4"'-N-dimethylaminobutoxy)phenyl]-7-hydroxy-3-(4'-hydroxyphenyl)-4-(trifluoromethyl)-2H-1-benzopyran
2-[4"-(4"'-N-diethylaminobutoxy)phenyl]-7-hydroxy-3-(4'-hydroxyphenyl)-4-(trifluoromethyl)-2H-1-benzopyran
7-hydroxy-3-(4'-hydroxyphenyl)-2-[4"-(4"'-N-methyl-N-phenethylaminobutoxy)phenyl]-4-(trifluoromethyl)-2H-1-benzopyran,
7-hydroxy-3-(4'-hydroxyphenyl)-2-{4"-[4"'-N-methyl-N-3""-phenylpropyl)aminobutoxy]phenyl}-4-(trifluoromethyl)-2H-1-benzopyran,
2-[4"-(4"'-N-butyl-N-ethylaminobutoxy)phenyl]-7-hydroxy-3-(4'-hydroxyphenyl)-4-(trifluoromethyl)-2H-1-benzopyran,
7-hydroxy-3-(4'-hydroxyphenyl)-2-[4"-(4"'-thiomorpholin-1-ylbutoxy)phenyl]-4-(trifluoromethyl)-2H-1-benzopyran,
7-hydroxy-3-(4'-hydroxyphenyl)-2-[4"-(4"'-pyrrolidin-1-ylbutoxy)phenyl]-4-(trifluoromethyl)-2H-1-benzopyran,
7-hydroxy-3-(4'-hydroxyphenyl)-2-[4"-(4"'-morpholin-1-ylbutoxy)phenyl]-4-(trifluoromethyl)-2H-1-benzopyran,
7-hydroxy-3-(4'-hydroxyphenyl)-2-[4"-(4"'-N-methyl-N-pentylaminobutoxy)phenyl]-4-(trifluoromethyl)-2H-1-benzopyran,
7-hydroxy-3-(4'-hydroxyphenyl)-2-[4"-(4"'-N-isobutyl-N-pentylaminobutoxy)phenyl]-4-(trifluoromethyl)-2H-1-benzopyran,
2-[4"-(4"'-N-benzyl-N-methylaminobutoxy)phenyl]-7-hydroxy-3-(4'-hydroxyphenyl)-4-(trifluoromethyl)-2H-1-benzopyran,
2-[4"-(4"'-N,N-di-n-propylaminobutoxy)phenyl]-7-hydroxy-3-(4'-hydroxyphenyl)-4-(trifluoromethyl)-2H-1-benzopyran,
2-[4"-(4"'-hexamethylenimin-1-ylbutoxy)phenyl]-7-hydroxy-3-(4'-hydroxyphenyl)-4-(trifluoromethyl)-2H-1-benzopyran,
2-[4"-(4"'-N-butyl-N-methylaminobutoxy)phenyl]-7-hydroxy-3-(4'-hydroxyphenyl)-4-(trifluoromethyl)-2H-1-benzopyran,
(+)-7-methoxy-3-(4'-methoxyphenyl)-2-[4"-(2"'-piperidin-1-ylethoxy)phenyl]-4-(trifluoromethyl)-2H-1-benzopyran,
(-)-7-methoxy-3-(4'-methoxyphenyl)-2-[4"-(2"'-piperidin-1-ylethoxy)phenyl]-4-(trifluoromethyl)-2H-1-benzopyran,
7-hydroxy-3-(4'-hydroxyphenyl)-2-[4"-(3"'-piperidin-1-yl-1-thiapropyl)phenyl]-4-(trifluoromethyl)-2H-1-benzopyran.

6. Compounds according to Claim 5 in the form of their racemates.

7. Compounds according to Claim 5 in the form of the separated optical isomers.

8. Compounds according to any of the preceding claims in the form of the hydrochloride.

9. Pharmaceutical products comprising at least one compound of the general formula I according to any of the preceding claims, and a pharmaceutically acceptable carrier.

10. Use of t he compounds of the general formula I according to any of the preceding claims for producing medicaments.

## Revendications

1. 4-Trifluorométhyl-2H-benzopyranes de formule générale I 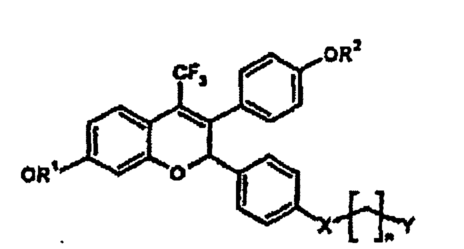 dans laquelle
R¹ et R² représentent, indépendamment l'un de l'autre, un atome d'hydrogène,
un groupe méthyle,
un groupe alkyle en C₂-C₅ linéaire ou ramifié,
un groupe alcanoyle linéaire ou ramifié ayant jusqu'à 5 atomes de carbone ou un groupe benzoyle,
X représente un atome d'oxygène ou de soufre ou un groupe méthylène,
n vaut 2, 3 ou 4,
Y représente un radical pipéridine,
pyrrolidine, héxaméthylène imino, diméthylamino, diéthylamino, dipropylamino, N-méthyl-N-phénéthylamino, N-méthyl-N-(3-phénylpropyl)amino, N-butyl-N-éthylamino, thiomorpholino, morpholino, N-méthyl-N-pentylamino, N -isobutyl-N-méthylamino, N-benzyl-N-méthylamino, N-butyl-N-méthylamino,
ou
un groupe -NR³R⁴, dans lequel R³ et R⁴ représentent, indépendamment l'un de l'autre, un atome d'hydrogène ; un groupe monofluorométhyle, difluorométhyle, trifluorométhyle, pentafluoroéthyle, 2,2,2-trifluoroéthyle, 4,4,4-trifluorobutyle, 3,3,4,4,4-pentafluorobutyle, 4,4,5,5,5-pentafluoropentyle, nonafluorobutyle,
ainsi que leurs sels d'addition physiologiquement acceptables avec des acides organiques et inorganiques.

2. Composés de formule générale I selon la revendication 1 sous forme de leurs racémates.

3. Composés de formule générale I selon la revendication 1 sous forme des isomères optiques séparés.

4. Composés de formule générale I selon l'une quelconque des revendications précédentes, dans laquelle R¹ et/ou R² sont un radical pivaloyle.

5. (+)-7-Hydroxy-3-(4'-hydroxyphényl)-2-[4"-(2"'-pipéridin-1-yléthoxy)phényl]-4-(trifluorométhyl)-2H-1-benzopyrane,
(-)-7-Hydroxy-3-(4'-hydroxyphényl)-2-[4"-(2"'-pipéridin-1-yléthoxy)phényl]-4-(trifluorométhyl)-2H-1-benzopyrane,
(+)-7-Pivaloyloxy-3-(4'-pivaloyloxyphényl)-4-trifluorométhyl-2-(4"-(2"'-pipéridinoéthoxy)phényl)-2H-benzopyrane,
(-)-7-Pivaloyloxy-3-(4'-pivaloyloxyphényl)-4-trifluorométhyl-2-(4"-(2"'-pipéridinoéthoxy)phényl)-2H-benzopyrane,
2-[4"-(2"'-Diméthylaminoéthoxy)phényl]-7-hydroxy-3-(4'-hydroxyphényl)-4-(trifluorométhyl)-2H-1-benzopyrane,
2-[4"-(2"'-Diéthylaminoéthoxy)phényl]-7-hydroxy-3-(4'-hydroxyphényl)-4-(trifluorométhyl)-2H-1-benzopyrane,
7-Hydroxy-3-(4'-hydroxyphényl)-2-[4"-(2"'-N-méthyl-N-phénéthylaminoéthoxy)phényl]-4-(trifluorométhyl)-2H-1-benzopyrane,
7-Hydroxy-3-(4'-hydroxyphényl)-2-{4"-(2"'-N-méthyl-N-(3""-phénylpropyl)aminoéthoxy]phényl}-4-(trifluoro-méthyl)-2H-1-benzopyrane,
2-[4"-(2"'-N-Butyl-N-éthylaminoéthoxy)phényl]-7-hydroxy-3-(4'-hydroxyphényl)-4-(trifluorométhyl)-2H-1-benzopyrane,
7-Hydroxy-3-(4'-hydroxyphényl)-2-[4"-(thiomorpholin-1-yléthoxy)phényl]-4-(trifluorométhyl)-2H-1-benzopyrane,
7-Hydroxy-3-(4'-hydroxyphényl)-2-[4"-(2"'-pyrrolidin-1-yléthoxy)phényl]-4-(trifluorométhyl)-2H-1-benzopyrane,
7-Hydroxy-3-(4'-hydroxyphényl)-2-[4"-(2"'-morpholin-1-yléthoxy)phényl]-4-(trifluorométhyl)-2H-1-benzopyrane,
7-Hydroxy-3-(4'-hydroxyphényl)-2-[4"-(2"'-N-méthyl-N-pentylaminoéthoxy)phényl]-4-(trifluorométhyl)-2H-1-benzopyrane,
7-Hydroxy-3-(4'-hydroxyphényl)-2-[4"-(2"'-N-isobutyl-N-méthylaminoéthoxy)phényl]-4-(trifluorométhyl)-2H-1-benzopyrane,
2-[4"-(2"'-N-Benzyl-N-méthylaminoéthoxy)phényl]-7-hydroxy-3-(4'-hydroxyphényl)-4-(trifluorométhyl)-2H-1-benzopyrane,
2-[4"-(2"'-N,N-Di-n-propylaminoéthoxy)phényl]-7-hydroxy-3-(4'-hydroxyphényl)-4-(trifluorométhyl)-2H-1-benzopyrane,
2-[4"-(2"'-Hexaméthylène-imin-1-yléthoxy)phényl]-7-hydroxy-3-(4'-hydroxyphényl)-4-(trifluorométhyl)-2H-1-benzopyrane,
2-[4"-(2"'-N-Butyl-N-méthylaminoéthoxy)phényl]-7-hydroxy-3-(4'-hydroxyphényl)-4-(trifluorométhyl)-2H-1-benzopyrane,
7-Hydroxy-3-(4'-hydroxyphényl)-2-[4"-(3"'-pipéridin-1-ylpropoxy)phényl]-4-(trifluorométhyl)-2H-1-benzopyrane,
2-[4"-(3"'-N-Diméthylaminopropoxy)phényl]-7-hydroxy-3-(4'-hydroxyphényl)-4-(trifluorométhyl)-2H-1-benzopyrane,
2-[4"-(3"'-N-Diéthylaminopropoxy)phényl]-7-hydroxy-3-(4'-hydroxyphényl)-4-(trifluorométhyl)-2H-1-benzopyrane,
7-Hydroxy-3-(4'-hydroxyphényl)-2-[4"-(3"'-N-méthyl-N-phenéthylaminopropoxy)phényl]-4-(trifluorométhyl)-2H-1-benzopyrane,
7-Hydroxy-3-(4'-hydroxyphényl)-2-{4"-[3"'-N-méthyl-N-(3""-phénylpropyl)aminopropoxy]phényl}-4-(trifluorométhyl)-2H-1-benzopyrane,
2-[4"-(3"'-N-Butyl-N-éthylaminopropoxy)phényl]-7-hydroxy-3-(4'-hydroxyphényl)-4-(trifluorométhyl)-2H-1-benzopyrane,
7-Hydroxy-3-(4'-hydroxyphényl)-2-[4"-(3"'-thiomorpholin-1-ylpropoxy)phényl]-4-(trifluorométhyl)-2H-1-benzopyrane,
7-Hydroxy-3-(4'-hydroxyphényl)-2-[4"-(3"'-pyrrolidin-1-ylpropoxy)phényl]-4-(trifluorométhyl)-2H-1-benzopyrane,
7-Hydroxy-3-(4'-hydroxyphényl)-2-[4"-(3"'-morpholin-1-ylpropoxy)phényl]-4-(trifluorométhyl)-2H-1-benzopyrane,
7-Hydroxy-3-(4'-hydroxyphényl)-2-[4"-(3"'-N-méthyl-N-pentylaminopropoxy)phényl]-4-(trifluorométhyl)-2H-1-benzopyrane,
7-Hydroxy-3-(4'-hydroxyphényl)-2-[4"-(3"'-N-isobutyl-N-méthylaminopropoxy)phényl]-4-(trifluorométhyl)-2H-1-benzopyrane,
2-[4"-(3"'-N-Benzyl-N-méthylaminopropoxy)phényl]-7-hydroxy-3-(4'-hydroxyphényl)-4-(trifluorométhyl)-2H-1-benzopyrane,
2-[4"-(3"'-N,N-Di-n-propylaminopropoxy)phényl]-7-hydroxy-3-(4'-hydroxyphényl)-4-(trifluorométhyl)-2H-1-benzopyrane,
2-[4"-(3"'-Hexaméthylène-imin-1-ylpropoxy)phényl]-7-hydroxy-3-(4'-hydroxyphényl)-4-(trifluorométhyl)-2H-1-benzopyrane,
2-[4"-(3"'-N-Butyl-N-méthylaminopropoxy)phényl]-7-hydroxy-3-(4'-hydroxyphényl)-4-(trifluorométhyl)-2H-1-benzopyrane,
7-Hydroxy-3-(4'-hydroxyphényl)-2-[4"-(4"'-pipéridin-1-ylbutoxy)phényl]-4-(trifluorométhyl)-2H-1-benzopyrane, 2-[4"-(4"'-N-Diméthylaminobutoxy)phényl]-7-hydroxy-3-(4'-hydroxyphényl)-4-(trifluorométhyl)-2H-1-benzopyrane,
2-[4"-(4"'-N-Diéthylaminobutoxy)phényl]-7-hydroxy-3-(4'-hydroxyphényl)-4-(trifluorométhyl)-2H-1-benzopyrane,
7-Hydroxy-3-(4'-hydroxyphényl)-2-[4"-(4"'-N-méthyl-N-phénéthylaminobutoxy)phényl]-4-(trifluorométhyl)-2H-1-benzopyrane,
7-Hydroxy-3-(4'-hydroxyphényl)-2-{4"-[4"'-N-méthyl-N-3""-phénylpropyl)aminobutoxy]phényl}-4-(trifluorométhyl)-2H-1-benzopyrane,
2-[4"-(4"'-N-Butyl-N-éthylaminobutoxy)phényl]-7-hydroxy-3-(4'-hydroxyphényl)-4-(trifluorométhyl)-2H-1-benzopyrane,
7-Hydroxy-3-(4'-hydroxyphényl)-2-[4"-(4"'-thiomorpholin-1-ylbutoxy)phényl]-4-(trifluorométhyl)-2H-1-benzopyrane,
7-Hydroxy-3-(4'-hydroxyphényl-2-[4"-(4"'-pyrrolidin-1-ylbutoxy)phényl]-4-(trifluorométhyl)-2H-1-benzopyrane,
7-Hydroxy-3-(4'-hydroxyphényl-2-[4"-(4"'-morpholin-1-ylbutoxy)phényl]-4-(trifluorométhyl)-2H-1-benzopyrane,
7-Hydroxy-3-(4'-hydroxyphényl-2-[4"-(4"'-N-méthyl-N-pentylaminobutoxy)phényl]-4-(trifluorométhyl)-2H-1-benzopyrane,
7-Hydroxy-3-(4'-hydroxyphényl-2-[4"-(4"'-N-isobutyl-N-méthylaminobutoxy)phényl]-4-(trifluorométhyl)-2H-1-benzopyrane,
2-[4"-(4"'-N-Benzyl-N-méthylaminobutoxy)phényl]-7-hydroxy-3-(4'-hydroxyphényl)-4-(trifluorométhyl)-2H-1-benzopyrane,
2-[4"-(4"'-N,N-Di-n-propylaminobutoxy)phényl]-7-hydroxy-3-(4'-hydroxyphényl)-4-(trifluorométhyl)-2H-1-benzopyrane,
2-[4"-(4"'-Hexaméthylène-imin-1-ylbutoxy)phényl]-7-hydroxy-3-(4'-hydroxyphényl)-4-(trifluorométhyl)-2H-1-benzopyrane,
2-[4"-(4"'-N-Butyl-N-méthylaminobutoxy)phényl]-7-hydroxy-3-(4'-hydroxyphényl)-4-(trifluorométhyl)-2H-1-benzopyrane,
(+)-7-Méthoxy-3-(4'-méthoxyphényl)-2-[4"-(2"'-pipéridin-1-yléthoxy)phényl]-4-(trifluorométhyl)-2H-1-benzopyrane,
(-)-7-Méthoxy-3-(4'-méthoxyphényl)-2-[4"-(2"'-pipéridin-1-yléthoxy)phényl]-4-(trifluorométhyl)-2H-1-benzopyrane,
7-Hydroxy-3-(4'-hydroxyphényl)-2-[4"-(3"'-pipéridin-1-yl-1-thiapropyl)phényl]-4-(trifluorométhyl)-2H-1-benzopyrane.

6. Composés selon la revendication 5 sous forme de leurs racémates.

7. Composés selon la revendication 5 sous forme des isomères optiques séparés.

8. Composés selon l'une quelconque des revendications précédentes sous forme du chlorhydrate.

9. Préparations pharmaceutiques contenant au moins un composé de formule générale I selon l'une quelconque des revendications précédentes ainsi qu'un support pharmaceutiquement acceptable.

10. Utilisation des composés de formule générale I selon l'une quelconque des revendications précédentes pour la préparation de médicaments.
